(19) Europäisches Patentamt
European Patent Office
Office européen des brevets

(11) Veröffentlichungsnummer : **0 410 359 B1**

# (12) EUROPÄISCHE PATENTSCHRIFT

(45) Veröffentlichungstag der Patentschrift :
**26.01.94 Patentblatt 94/04**

(21) Anmeldenummer : **90114102.8**

(22) Anmeldetag : **23.07.90**

(51) Int. Cl.$^5$ : **C07C 217/22,** C07C 217/46, C07C 213/08, C07C 213/02, C07C 217/20, C07C 217/18, C07C 49/84, A61K 31/135

## (54) Substituierte Aminoalkoxybenzolderivate.

(30) Priorität : **27.07.89 CH 2798/89**
**08.05.90 CH 1553/90**

(43) Veröffentlichungstag der Anmeldung :
**30.01.91 Patentblatt 91/05**

(45) Bekanntmachung des Hinweises auf die Patenterteilung :
**26.01.94 Patentblatt 94/04**

(84) Benannte Vertragsstaaten :
**AT BE CH DE DK ES FR GB GR IT LI LU NL SE**

(56) Entgegenhaltungen :
**EP-A- 0 114 410**
**US-A- 1 894 865**
**US-A- 2 668 813**
**US-A- 2 668 850**
**US-A- 3 312 696**
**US-A- 3 560 567**

(73) Patentinhaber : **F. HOFFMANN-LA ROCHE AG**
**Postfach 3255**
**CH-4002 Basel (CH)**

(72) Erfinder : **Guerry, Philippe, Dr.**
**Burgfelderstrasse 30**
**CH-4055 Basel (CH)**
Erfinder : **Jolidon, Synèse, Dr.**
**Schillerstrasse 5**
**CH-4127 Birsfelden (CH)**
Erfinder : **Zurflüh, René, Dr.**
**Dachslenbergstrasse 54**
**CH-8180 Bülach (CH)**

(74) Vertreter : **Lederer, Franz, Dr. et al**
**Lederer, Keller & Riederer, Patentanwälte,**
**Lucile-Grahn-Strasse 22**
**D-81675 München (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von Verbindungen der allgemeinen Formel

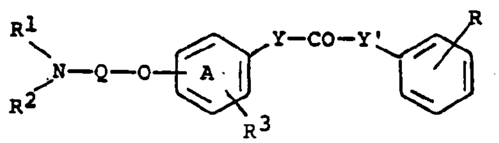

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q-O- an die 3- oder 4-Stellung des mit A bezeichneten Ringes gebunden ist und das Symbol R bedeutet, dass der Ring unsubstituiert oder durch Halogene Trifluormethyl, Cyano, Nitro, niederes Alkyl und/oder niederes Alkoxy einfach oder mehrfach substituiert ist,

und ihrer pharmazeutisch annehmbaren Säureadditionssalze zur Bekämpfung oder Verhütung von fungalen Infektionen, insbesondere von topischen oder systemischen Infektionen, welche durch pathogene Pilze verursacht werden, und zur Herstellung von antifungal wirksamen Mitteln. Die Verbindungen der Formel I besitzen nicht nur eine ausgeprägte antifungale Wirkung, sondern sie zeigen auch synergistische Effekte in Kombination mit anderen, bekannten, antifungal wirksamen Substanzen, welche die Sterol-Biosynthese hemmen, wie Ketoconazol und Terbinafin.

In einem speziellen Aspekt betrifft die vorliegende Erfindung die Verwendung von 4-[(4-(Dimethylamino)butyl)oxy]benzophenon für die soeben erwähnten Zwecke.

Weitere Gegenstände der vorliegenden Erfindung sind Verbindungen der allgemeinen Formel

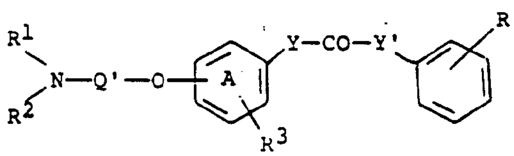

Ia

worin Q' Alkylen mit 5 bis 11 Kohlenstoffatomen und mindestens 5 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen bedeutet, und $R^1$, $R^2$, $R^3$, A, Y und Y' obige Bedeutung besitzen,

und das Symbol R bedeutet, dass der Ring unsubstituiert oder durch Trifluormethyl, Cyano, niederes Alkyl und/oder niederes Alkoxy einfach oder mehrfach substituiert ist.

und ihre pharmazeutisch annehmbaren Säureadditionssalze zur Anwendung als therapeutische Wirkstoffe, insbesondere als antifungal wirksame Stoffe, entsprechende Arzneimittel auf der Basis dieser Verbindungen der Formel Ia und, sofern Y und Y' nicht gleichzeitig eine direkte Bindung bedeuten, wenn Q' Alkylen mit 5 Kohlenstoffatomen und $R^1$ und $R^2$ gleichzeitig niederes Alkyl mit mehr als 2 Kohlenstoffatomen bedeuten, die Verbindungen der Formel Ia als solche, deren Herstellung und Zwischenprodukte zu deren Herstellung.

Verbindungen der Formel I, worin Q unverzweigtes Alkylen mit 4 Kohlenstoffatomen bedeutet, gehören einer an sich bekannten Stoffklasse an. In der U.S. Patentschrift Nr. 3,864,501 werden derartige Verbindungen als Mittel zur Verbesserung der Färbung von Früchten und Gemüsen beschrieben. In der U.S. Patentschrift Nr. 3,312,696 werden derartige Verbindungen als Koronardilatantien beschrieben. In der Europäischen Patentpublikation Nr. 115,080 werden derartige Verbindungen als Zwischenprodukte zur Herstellung von Wirkstoffen gegen Alkoholvergiftungen beschrieben.

Verbindungen der Formel I, worin Y und Y' je eine direkte Bindung, $R^1$ und $R^2$ je niederes Alkyl mit mehr als 2 Kohlenstoffatomen und Q unverzweigtes Alkylen mit 5 Kohlenstoffatomen bedeuten, gehören ebenfalls einer an sich bekannten Stoffklasse an. In der Europäischen Patentpublikation Nr. 114,410 werden derartige Verbindungen als Zwischenprodukte zur Herstellung von Wirkstoffen gegen Alkoholvergiftungen beschrieben. Die US-Patentschrift 3 560 567 beschreibt Benzophenonderivate der obigen Formel in denen R p-Brom, p-Jod oder p-Nitro ist. Diese Verbindungen sind als gegen Tuberkulose wirksam beschrieben.

Der Ausdruck "nieder" bezeichnet Reste und Verbindungen mit höchstens sieben, vorzugsweise höchstens vier Kohlenstoffatomen. Der Ausdruck "Alkyl" bezeichnet geradkettige oder verzweigte, gesättigte Kohlenwasserstoffreste, wie Methyl, Aethyl, Propyl, Isopropyl und t-Butyl. Der Ausdruck "Alkenyl" bezeichnet geradkettige oder verzweigte Kohlenwasserstoffreste mit einer olefinischen Doppelbindung, wie Allyl und 2-Butenyl. Der Ausdruck "Alkoxy" bezeichnet über ein Sauerstoffatom gebundene Alkylgruppen, wie Methoxy und Aethoxy. Der Ausdruck "Alkylen" bezeichnet geradkettige oder verzweigte, gesättigte Kohlenwasserstoffreste mit zwei freien Valenzen, wie Dimethylen, Trimethylen oder Tetramethylen. Der Ausdruck "Alkenylen" bezeichnet geradkettige oder verzweigte Kohlenwasserstoffreste mit mindestens einer olefinischen Doppelbindung und zwei freien Valenzen, wie 2-Buten-1,4-diyl. Der Ausdruck "Halogen" bezeichnet die vier Formen Fluor, Chlor, Brom und Jod.

Der weiter unten verwendete Ausdruck "Abgangsgruppe" bezeichnet vorzugsweise Halogenatome, insbesondere Chlor, Brom und Jod, und niedere Alkyl- und Arylsulfonyloxygruppen, wie Methylsulfonyloxy, Benzolsulfonyloxy, p-Toluolsulfonyloxy und p-Chlorbenzolsulfonyloxy.

$R^1$ und $R^2$ bedeuten vorzugsweise je $C_{1-4}$-Alkyl, $C_{3-4}$-Alkenyl oder zusammen $C_{3-4}$-Alkylen. Q' bedeutet vorzugsweise unverzweigtes Alkylen mit 5 bis 7 Kohlenstoffatomen. Die Gruppe $R^1R^2N$-Q'-O- ist vorzugsweise an die 4-Stellung des mit A bezeichneten Ringes gebunden. Y bedeutet vorzugsweise eine direkte Bindung oder die Gruppe $-CH_2-$, insbesondere eine direkte Bindung. Y' bedeutet vorzugsweise eine direkte Bindung oder die Gruppe $-CH_2-$, $-CH_2CH_2-$ oder -CH=CH-, insbesondere eine direkte Bindung oder die Gruppe $CH_2-$. Das Symbol R bedeutet vorzugsweise, dass der Ring unsubstituiert oder durch niederes Alkyl substituiert, vorzugsweise mono- oder disubstituiert ist.

Im Rahmen der vorliegenden Erfindung besonders bevorzugte, neue Verbindungen der Formel Ia sind:

4-[(6-(Dimethylamino)hexyl)oxy]-2-phenylacetophenon,
4-[(6-(Dimethylamino)hexyl)oxy]benzophenon,
4'-[(6-(Diäthylamino)hexyl)oxy]-3-phenylpropiophenon,
4'-[(6-(Dimethylamino)hexyl)oxy]-3-phenylpropiophenon,
(E)-4'-[[6-(Dimethylamino)hexyl]oxy]-3-phenylacrylophenon,
4-[(6-Dimethylamino)hexyl)oxy]benzophenon,
4-[(6-(1-Azetidinyl)hexyl)oxy]benzophenon,
4-[(6-(1-Pyrrolidinyl)hexyl)oxy]benzophenon,
2-[4-[[6-(Dimethylamino)hexyl]oxy]phenyl]acetophenon,
4-[(7-(Dimethylamino)heptyl)oxy]benzophenon,
4-[(5-(Dimethylamino)pentyl)oxy]benzophenon,
4-[[6-(Allylmethylamino)hexyl]oxy]-2-phenylacetophenon,
trans-4-[[4-(Allylmethylamino)-2-butenyl]oxy]benzophenon,
4-[[6-(Allylmethylamino)hexyl]oxy]-3-methylbenzophenon,

und

4-[[6-(Allylmethylamino)hexyl]oxy]-3-chlor-benzophenon.

Die neuen Verbindungen der Formel Ia und ihre pharmazeutisch annehmbaren Säureadditionssalze können erfindungsgemäss hergestellt werden, indem man

a) eine Verbindung der allgemeinen Formel

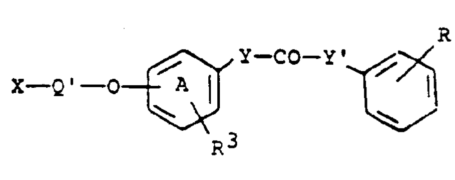

II

worin X eine Abgangsgruppe bedeutet, und A, Q', Y, Y', $R^3$ und R obige Bedeutung besitzen,

mit einem Amin der allgemeinen Formel $HNR^1R^2$, worin $R^1$ und $R^2$ obige Bedeutung besitzen, umsetzt, oder

b) eine Verbindung der allgemeinen Formel

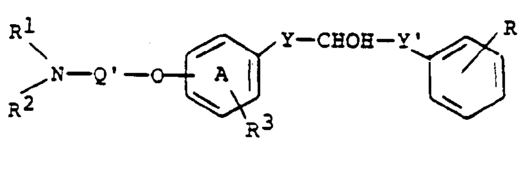

III

worin A, $R^1$, $R^2$, $R^3$, Q', Y, Y' und R obige Bedeutung besitzen,

oxidiert, oder

c) eine Verbindung der allgemeinen Formel

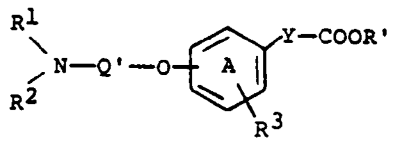

IV

worin R' niederes Alkyl bedeutet, und A, $R^1$, $R^2$, $R^3$, Q' und Y obige Bedeutung besitzen, mit einer Verbindung der allgemeinen Formel

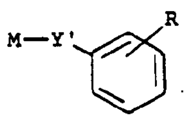

V

worin M -MgCl, -MgBr, -MgJ oder -Li bedeutet, und Y' und R obige Bedeutung besitzen, umsetzt, oder

d) eine Verbindung der allgemeinen Formel

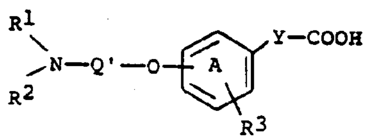

VI

worin A, $R^1$, $R^2$, $R^3$, Q' und Y obige Bedeutung besitzen, in Form eines reaktiven Derivates in Gegenwart einer Lewis-Säure mit einer Verbindung der allgemeinen Formel

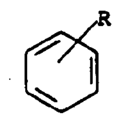

VII

worin R obige Bedeutung besitzt, umsetzt, oder

e) eine Verbindung der allgemeinen Formel

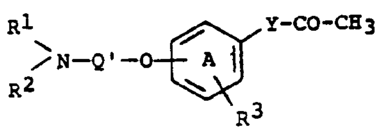

VIII

worin A, $R^1$, $R^2$, $R^3$, Q' und Y obige Bedeutung besitzen, in Gegenwart einer Base mit einer Verbindung der allgemeinen Formel

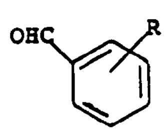

IX

worin R obige Bedeutung besitzt, umsetzt, oder

f) eine Verbindung der allgemeinen Formel

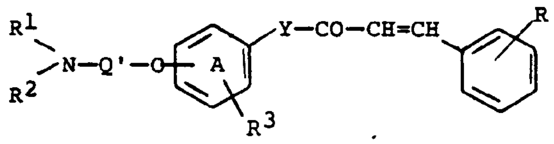

I'

worin A, $R^1$,$R^2$, $R^3$ Q', Y und R obige Bedeutung besitzen,
hydriert, oder
g) eine Verbindung der allgemeinen Formel

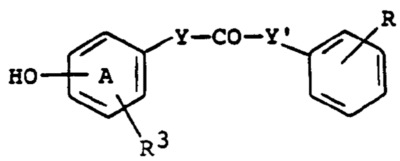

X

worin A, R, $R^3$, Y und Y' obige Bedeutung besitzen, in Gegenwart von Triphenylphosphin und einem Azodicarbonsäure-di(niederem Alkylester) mit einer Verbindung der allgemeinen Formel

$R^1R^2N$-Q'-OH XI

worin $R^1$, $R^2$ und Q' obige Bedeutung besitzen, umsetzt, und
h) eine erhaltene Verbindung der Formel Ia erwünschtenfalls in ein pharmazeutisch annehmbares Säureadditionssalz überführt.

Die Umsetzung einer Verbindung der Formel II mit einem Amin der Formel $HNR^1R^2$ gemäss Verfahrensvariante a) kann nach an sich bekannten und jedem Fachmann geläufigen Methoden durchgeführt werden. Die Reaktion wird vorzugsweise in einem polaren Lösungsmittel und in Gegenwart einer Base als säurebindendes Mittel in einem Temperaturbereich von etwa 0°C bis etwa 150°C durchgeführt. Geeignete Lösungsmittel sind beispielsweise niedere Alkohole, wie Methanol und Aethanol, und niedere Dialkylketone, wie Aceton. Geeignete Basen sind beispielsweise überschüssiges Amin der Formel $HNR^1R^2$, tertiäre Amine, wie Triäthylamin, und anorganische Basen, wie Alkalimetallcarbonate, -hydroxide und -alkoholate.

Die Oxidation einer Verbindung der Formel III gemäss Verfahrensvariante b) kann nach an sich bekannten und jedem Fachmann geläufigen Methoden durchgeführt werden. Die Reaktion wird vorzugsweise in einem inerten Lösungsmittel und in Gegenwart eines Oxidationsmittels in einem Temperaturbereich von etwa -80°C bis etwa Raumtemperatur durchgeführt. Geeignete Lösungsmittel sind beispielsweise chlorierte, niedere Kohlenwasserstoffe, wie Methylenchlorid und Chloroform. Geeignete Oxidationsmittel sind beispielsweise Mangandioxid oder Mischungen von Dimethylsulfoxid mit Oxalylchlorid, Dicyclohexylcarbodiimid oder Acetanhydrid und einem tertiären Amin, wie Triäthylamin.

Die Umsetzung einer Verbindung der Formel IV mit einer Verbindung der Formel V gemäss Verfahrensvariante c) kann nach an sich bekannten und jedem Fachmann geläufigen Methoden durchgeführt werden. Die Reaktion wird vorzugsweise in einem inerten Lösungsmittel und in einem Temperaturbereich von etwa -80°C bis etwa Raumtemperatur durchgeführt. Geeignete Lösungsmittel sind beispielsweise offenkettige und cyclische Aether, wie Diäthyläther, Methyl-t-butyläther und Tetrahydrofuran, und Mischungen davon.

Die Umsetzung eines reaktiven Derivates einer Verbindung der Formel VI mit einer Verbindung der Formel VII gemäss Verfahrensvariante d) kann nach an sich bekannten und jedem Fachmann geläufigen Methoden durchgeführt werden. Die Reaktion wird vorzugsweise in einem inerten Lösungsmittel und in Gegenwart einer Lewis-Säure in einem Temperaturbereich von etwa 0°C bis etwa 100°C durchgeführt. Geeignete Lösungsmittel sind beispielsweise halogenierte niedere Kohlenwasserstoffe, wie Methylenchlorid, Chloroform und Aethylenchlorid, Nitrobenzol, Schwefelkohlenstoff und überschüssige Verbindung der Formel VII. Als Lewis-Säure verwendet man vorzugsweise Aluminiumchlorid. Geeignete reaktive Derivate von Verbindungen der Formel VI sind beispielsweise die entsprechenden Carbonsäurechloride.

Die Umsetzung einer Verbindung der Formel VIII mit einer Verbindung der Formel IX in Gegenwart einer Base gemäss Verfahrensvariante e) kann nach an sich bekannten und jedem Fachmann geläufigen Methoden durchgeführt werden. Die Reaktion wird vorzugsweise in einem polaren Lösungsmittel und in einem Temperaturbereich von etwa 0°C bis etwa 60°C durchgeführt. Geeignete Lösungsmittel sind beispielsweise niedere Alkohole, wie Methanol und Aethanol, und Mischungen davon mit Wasser. Als Basen verwendet man vorzugsweise Alkalimetallcarbonate und -hydroxide, wie Kaliumcarbonat und Natriumhydroxid.

Die Hydrierung einer Verbindung der Formel I' gemäss Verfahrensvariante f) kann nach an sich bekannten und jedem Fachmann geläufigen Methoden durchgeführt werden. Die Reaktion wird vorzugsweise in einem polaren Lösungsmittel mit elementarem Wasserstoff in Gegenwart eines geeigneten Hydrierungskatalysators und in einem Temperaturbereich von etwa 0°C bis etwa Raumtemperatur durchgeführt. Geeignete Lösungsmittel sind beispielsweise niedere Alkohole, wie Methanol und Aethanol. Geeignete Katalysatoren sind beispielsweise Palladium oder Platin auf Kohle, Platinoxid oder Raney-Nickel.

Bei der Umsetzung einer Verbindung der Formel X mit einer Verbindung der Formel XI gemäss Verfahrensvariante g) handelt es sich ebenfalls um eine an sich bekannte Methode, nämlich um die sogenannte Mitsunobu-Kopplung. Sie wird vorzugsweise in einem inerten organischen Lösungsmittel und in einem Temperaturbereich von etwa 0°C bis zur Siedetemperatur der Reaktionsmischung durchgeführt. Geeignete Lösungsmittel sind beispielsweise chlorierte, niedere Kohlenwasserstoffe, wie Methylenchlorid und Chloroform, und offenkettige und cyclische Aether, wie Diäthyläther, Methyl-t-butyläther und Tetrahydrofuran, und Mischungen davon.

Die Herstellung von pharmazeutisch annehmbaren Säureadditionssalzen von Verbindungen der Formel Ia gemäss Verfahrensvariante h) kann nach an sich bekannten und jedem Fachmann geläufigen Methoden durchgeführt werden. Es kommen dabei Salze mit pharmazeutisch annehmbaren anorganischen und organischen Säuren in Betracht. Bevorzugte Säureadditionssalze sind die Hydrochloride, Hydrobromide, Sulfate, Nitrate, Citrate, Acetate, Succinate, Fumarate, Methansulfonate und die p-Toluolsulfonate.

Die bekannten Verbindungen der Formel I und ihre pharmazeutisch annehmbaren Säureadditionssalze können ebenfalls nach den obigen Verfahren a)-h) hergestellt werden. Die entsprechenden Ausgangsstoffe können, wie nachstehend für die Ausgangsstoffe für die neuen Verbindungen der Formel Ia beschrieben, hergestellt werden.

Die als Ausgangsstoffe verwendeten Verbindungen der Formeln II, III, IV, VI und VIII können beispielsweise gemäss den nachfolgenden Reaktionsschemata I-IV und den nachfolgenden Beschreibungen der verschiedenen Reaktionen hergestellt werden. Die übrigen als Ausgangsstoffe verwendeten Verbindungen gehören an sich bekannten Stoffklassen an. In diesen Reaktionsschemata haben $R^1$, $R^2$, $R^3$, R, R', A, M, Q', X, Y und Y' die weiter oben für die neuen Verbindungen der Formel Ia bzw. deren Herstellung angegebene Bedeutung.

Reaktionsschema I

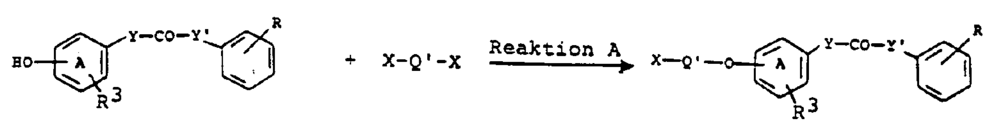


X XII II

## Reaktionsschema II

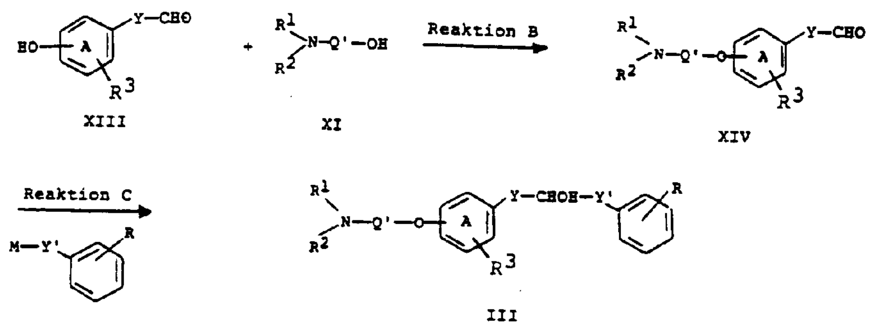


XIII XI XIV V III

## Reaktionsschema III

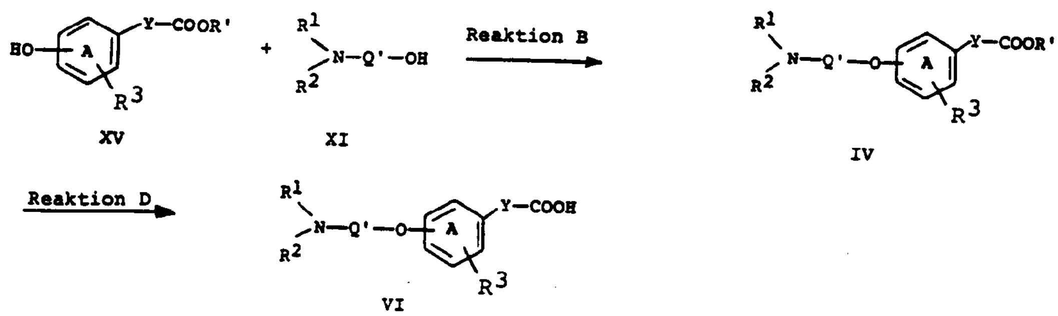


XV XI IV VI

## Reaktionsschema IV

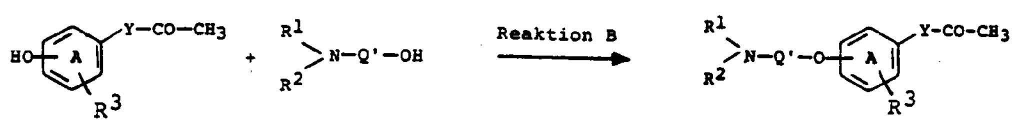


XVI XI VIII

Reaktion A

Diese Reaktion kann nach an sich bekannten und jedem Fachmann geläufigen Methoden durchgeführt werden und wird vorzugsweise in einem polaren Lösungsmittel und in Gegenwart einer Base in einem Temperaturbereich von etwa 0°C bis etwa 150°C durchgeführt. Geeignete Lösungsmittel sind beispielsweise niedere Alkohole, wie Methanol und Aethanol, und niedere Dialkylketone, wie Aceton. Geeignete Basen sind beispielsweise Alkalimetallcarbonate, -hydroxide, -alkoholate und -hydride.

Die Reaktion A kann jedoch auch in einem Zweiphasensystem in Gegenwart eines Phasentransferkatalysators, beispielsweise eines quaternären Ammoniumsalzes, durchgeführt werden. Als wässrige Phase verwendet man vorzugsweise eine wässrige Lauge, wie Natronlauge, und als organische Phase einen halogenierten niederen Kohlenwasserstoff, wie Methylenchlorid, oder einen aromatischen Kohlenwasserstoff, wie Toluol. Die Verbindung der Formel XII wird vorzugsweise im Ueberschuss verwendet, wobei man vorzugsweise 2 bis 4 Mol-Aequivalente der Verbindung der Formel XII verwendet.

Reaktion B

Bei dieser Reaktion handelt es sich um die weiter oben im Zusammenhang mit der Verfahrensvariante g) beschriebene Mitsunobu-Kopplung. Sie wird vorzugsweise in einem inerten organischen Lösungsmittel und in einem Temperaturbereich von etwa 0°C bis zur Siedetemperatur des Reaktionsgemisches durchgeführt. Geeignete Lösungsmittel sind beispielsweise chlorierte, niedere Kohlenwasserstoffe, wie Methylenchlorid und Chloroform, und offenkettige und cyclische Aether, wie Diäthyläther, Methyl-t-butyläther und Tetrahydrofuran, und Mischungen davon.

Reaktion C

Die Umsetzung einer Verbindung der Formel XIV mit einer Verbindung der Formel V kann nach an sich bekannten und jedem Fachmann geläufigen Methoden durchgeführt werden. Die Reaktion wird vorzugsweise in einem inerten Lösungsmittel und in einem Temperaturbereich von etwa -80°C bis etwa Raumtemperatur durchgeführt. Geeignete Lösungsmittel sind beispielsweise offenkettige und cyclische Aether, wie Diäthyläther, Methyl-t-butyläther und Tetrahydrofuran, und Mischungen davon.

Reaktion D

Bei dieser Reaktion handelt es sich um eine Hydrolyse. Diese kann nach an sich bekannten und jedem Fachmann geläufigen Methoden durchgeführt werden und wird vorzugsweise durch Behandeln mit einem Alkalimetallhydroxid, wie Natrium- und Kaliumhydroxid, oder mit einer Mineralsäure, wie Salzsäure und Bromwasserstoffsäure, in einem polaren Lösungsmittel und in einem Temperaturbereich von etwa 0°C bis etwa 100°C durchgeführt. Geeignete Lösungsmittel sind beispielsweise Mischungen von niederen Alkoholen, wie Methanol und Aethanol, und mit Wasser mischbaren offenkettigen und cyclischen Aethern, wie Tetrahydrofuran, mit Wasser.

Wie bereits erwähnt besitzen die Verbindungen der Formel I und ihre pharmazeutisch annehmbaren Säureadditionssalze wertvolle antifungale Eigenschaften. Sie sind wirksam gegen eine Vielzahl von pathogenen Pilzen, die topische und systemische Infektionen hervorrufen, wie Candida albicans und Histoplasma capsulatum. Die 2,3-Epoxysqualen-Lanosterol-Cyclase, ein Enzym involviert in der Sterolbiosynthese der eucaryotischen Zelle, ist ein essentielles Enzym für die Pilze. So ist z.B. ein S.cerevisiae Stamm, in welchem dieses Enzym fehlt nicht lebensfähig [F. Karst & F. Lacroute, Molec. Gen. Genet. 154, 269 (1977)]. Die hemmende Wirkung der Verbindungen der Formel I auf obengenanntes Enzym aus C.albicans wurde als Mass für die antifungale Wirkung genommen. Die Hemmung kann, beispielsweise, mittels der nachfolgend beschriebenen Methode gemessen werden.

Bestimmung des IC50-Wertes für die Hemmung der 2,3-Epoxysqualen-Lanosterol-Cyclase von Candida albicans

Die Zellen einer Kultur von Candida albicans am Ende der logarithmischen Wachstumsphase werden gesammelt und mit 100mM Phosphatpuffer (pH = 6,9). Digestionspuffer und 50mM Phosphatpuffer (pH = 7,4), welcher 1M Mannitol und 5mM DTT enthält, gewaschen.

1,0 g dieser Zellen werden in 5 ml Digestionspuffer aufgeschlämmt, mit 1 mg Zymolase 100T (Seikagaku Kogyo, Japan) und 12,5 µl β-Mercaptoäthanol versetzt und während 30 Minuten bei 30°C inkubiert. Die entstehenden Protoplasten werden durch Zentrifugation (10 Minuten bei 2500 g) isoliert und anschliessend durch Zugabe von 2 ml 100mM Phosphatpuffer (pH = 6,9) zum Platzen gebracht. Durch erneute Zentrifugation (10 Minuten bei 10000 g) erhält man einen zellfreien Extrakt (CFE) als Ueberstand. Dieser wird auf 10 mg Protein pro ml verdünnt, und der pH wird auf 6,9 gebracht.

Die Aktivität der 2,3-Epoxysqualen-Lanosterol-Cyclase im CFE wird durch die Umsetzung von $^{14}C$-Squalen-Epoxid in Gegenwart von n-Decylpentaoxyäthylen als Detergens gemessen. Titration mit angemessenen Mengen der Testsubstanz erlauben die Bestimmung des $IC_{50}$-Wertes (Konzentration der Testsubstanz, welche die Enzymaktivität um die Hälfte verringert).

Der Versuch wird wie folgt durchgeführt:

Durch Ultraschallbehandlung bereitet man eine 250µM Lösung von $^{14}C$-Squalen-Epoxid in 100mM Phosphatpuffer (pH = 6,9) unter Zusatz von 1% n-Decylpentaoxyäthylen vor. 100 µl dieser Lösung wird mit 20 µl einer Lösung der Testsubstanz in Dimethylsulfoxid (bzw. 20 µl reinem Dimethylsulfoxid als Kontrolle) versetzt. Nach Zugabe von 880 µl CFE wird die gut gemischte Lösung unter Schütteln während 1 Stunde bei 30° inkubiert. Anschliessend wird durch Zugabe von 500 µl 15-proz. Kaliumhydroxid in 90-proz. Aethanol die Reaktion gestoppt.

Das Gemisch wird zweimal mit 1 ml n-Hexan extrahiert, das Hexan wird abgedampft und der Lipid-Rückstand wird in 200 µl Diäthyläther aufgenommen. Nach Dünnschichtchromatographie an Kieselgel mit Methylenchlorid als Laufmittel werden die Platten mit einem Radioaktivitäts-Dünnschicht-Scanner untersucht.

Unter den verwendeten Bedingungen wird ausschliesslich Lanosterol als radioaktives Produkt gefunden. Dessen Menge wird mit der Menge radioaktivem Lanosterol in der Kontrolle verglichen.

Die $IC_{50}$-Werte werden graphisch ermittelt und in µg Testsubstanz pro ml angegeben. Die nachfolgende Tabelle I enthält für repräsentative Vertreter der durch die Formel I definierten Verbindungsklasse die in obigem Versuch ermittelten $IC_{50}$-Werte, sowie Angaben über die akute Toxizität bei subkutaner Verabreichung an Mäusen ($DL_{50}$ in mg/kg).

Tabelle I

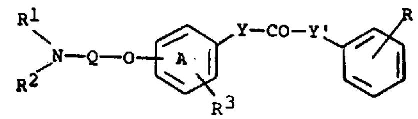

| Nr. | $R^1$ | $R^2$ | $R^3$ | Q (Pos.) | Y | Y' | R | $IC_{50}$ in µg/ml | $DL_{50}$ in mg/kg s.c. |
|---|---|---|---|---|---|---|---|---|---|
| 1 | $-CH_3$ | $-CH_3$ | H | $-(CH_2)_4-(4)$ | - | - | H | 0,85 | >4000 |
| 2 | $-CH_3$ | $-CH_3$ | H | $-(CH_2)_5-(4)$ | - | - | H | 0,16 | |
| 3 | $-CH_3$ | $-CH_3$ | H | $-(CH_2)_6-(4)$ | - | - | H | 0,15 | |
| 4 | $-CH_2CH_3$ | $-CH_2CH_3$ | H | $-(CH_2)_6-(3)$ | - | $-CH_2CH_2-$ | H | 0,60 | |
| 5 | $-CH_3$ | $-CH_3$ | H | $-(CH_2)_6-(4)$ | - | -CH=CH- | H | 0,60 | |
| 6 | $-CH_3$ | $-CH_3$ | H | $-(CH_2)_6-(4)$ | - | $-CH_2-$ | H | 0,065 | |
| 7 | $-CH_2CH_3$ | $-CH_2CH_3$ | H | $-(CH_2)_6-(4)$ | - | $-CH_2CH_2-$ | H | 0,27 | |
| 8 | $-CH_3$ | $-CH_3$ | H | $-(CH_2)_6-(4)$ | - | $-CH_2CH_2-$ | H | 0,35 | |
| 9 | $-CH_2CH_3$ | $-CH_2CH_3$ | H | $-(CH_2)_6-(4)$ | - | - | H | 0,36 | |
| 10 | $-CH_3$ | $-CH_3$ | H | $-(CH_2)_6-(3)$ | - | -CH=CH- | H | 2,30 | |
| 11 | $-CH_3$ | $-CH_3$ | H | $-(CH_2)_6-(3)$ | - | $-CH_2CH_2-$ | H | 1,00 | |
| 12 | $-CH_3$ | $-CH_3$ | H | $-(CH_2)_6-(4)$ | - | - | 4-Fluor | 0,18 | |
| 13 | $-CH_3$ | $-CH_3$ | H | $-(CH_2)_6-(3)$ | - | - | H | 2,00 | |
| 14 | $-CH_2CH_3$ | $-CH_2CH_3$ | H | $-(CH_2)_6-(4)$ | - | -C≡C- | H | 3,10 | |
| 15 | $-CH_3$ | $-CH_3$ | H | $-(CH_2)_6-(4)$ | - | - | Pentafluor | 0,89 | |
| 16 | $-(CH_2)_2CH_3$ | $-(CH_2)_2CH_3$ | H | $-(CH_2)_6-(4)$ | - | - | H | 0,92 | |
| 17 | $-(CH_2)_3-$ | | H | $-(CH_2)_6-(4)$ | - | - | H | 0,60 | |
| 18 | $-CH_3$ | $-CH_3$ | H | $-(CH_2)_7-(4)$ | - | - | H | 0,12 | |
| 19 | $-CH_3$ | $-CH_3$ | H | $-(CH_2)_8-(4)$ | - | - | H | 0,42 | |
| 20 | $-CH_3$ | $-CH_3$ | H | $-(CH_2)_9-(4)$ | - | - | H | 1,60 | |

Tabelle I (Fortsetzung)

| | | | | | | | | | |
|---|---|---|---|---|---|---|---|---|---|
| 21 | $-CH_3$ | $-CH_3$ | H | $-(CH_2)_{10}-(4)$ | - | - | H | 2,00 | |
| 22 | $-(CH_2)_4-$ | | H | $-(CH_2)_6-(4)$ | - | - | H | 0,34 | |
| 23 | $-CH_3$ | $-CH_3$ | H | $-(CH_2)_6-(4)$ | $-CH_2-$ | - | H | 0,19 | |
| 24 | $-CH_3$ | H | H | $-(CH_2)_6-(4)$ | - | - | H | 0,30 | |
| 25 | $-CH_3$ | $-CH_3$ | $-CH_3(3)$ | $-(CH_2)_6-(4)$ | - | - | H | 0,19 | |
| 26 | $-CH_3$ | $-CH_3$ | H | $-(CH_2)_6-(4)$ | - | - | 2,3-Dimethyl | 0,42 | |
| 27 | $-CH_3$ | $-CH_3$ | H | $-(CH_2)_6-(4)$ | - | - | 2,4-Dichlor | 0,26 | |
| 28 | $-CH_3$ | $-CH_3$ | H | $-(CH_2)_6-(4)$ | - | - | 3,5-Dichlor | 1,50 | |
| 29 | $-CH_3$ | $-CH_3$ | H | $-(CH_2)_6-(4)$ | - | - | 4-Nitro | 0,28 | |
| 30 | $-CH_3$ | $-CH_3$ | -Cl(3) | $-(CH_2)_6-(4)$ | - | - | 2,4-Dichlor | 0,10 | |
| 31 | $-CH_2-CH=CH_2$ | $-CH_3$ | H | $-(CH_2)_6-(4)$ | - | - | H | 0,044 | 1000-2000 |
| 32 | $-CH_2-CH=CH_2$ | $-CH_3$ | H | $-(CH_2)_6-(4)$ | - | $-CH_2-$ | H | 0,035 | |
| 33 | $-CH_2-CH=CH_2$ | $-CH_3$ | H | $-(CH_2)_6-(4)$ | - | - | 4-Fluor | 0,031 | |
| 34 | $-CH_2-CH=CH_2$ | $-CH_3$ | $-CH_3(3)$ | $-(CH_2)_6-(4)$ | - | - | H | 0,032 | |
| 35 | $-CH_2-CH=CH_2$ | $-CH_3$ | H | $-(CH_2)_6-(4)$ | - | - | 2,3-Dimethyl | 0,085 | |
| 36 | $-CH_2-CH=CH_2$ | $-CH_3$ | H | $-(CH_2)_6-(4)$ | - | - | 2,4-Dichlor | 0,055 | |
| 37 | $-CH_2-CH=CH_2$ | $-CH_3$ | H | $-(CH_2)_6-(4)$ | - | - | 3,5-Dichlor | 0,34 | |
| 38 | $-CH_2-CH=CH_2$ | $-CH_3$ | H | $-(CH_2)_6-(4)$ | - | - | 4-Nitro | 0,04 | |
| 39 | $-CH_2-CH=CH_2$ | $-CH_3$ | -Cl(3) | $-(CH_2)_6-(4)$ | - | - | H | 0,025 | |
| 40 | $-CH_2-CH=CH_2$ | $-CH_3$ | -Cl(3) | $-(CH_2)_6-(4)$ | - | - | 2,4-Dichlor | 0,026 | |
| 41 | $-CH_2-CH=CH_2$ | $-CH_3$ | H | $-CH_2-CH=CH-CH_2-(4)$ | - | - | H | 0,02 | |
| 42 | $-CH_2-CH=CH_2$ | $-CH_3$ | H | $-CH_2-CH=CH-CH_2-(4)$ | - | - | 2,4-Dichlor | 0,007 | |
| 43 | $-CH_3$ | $-CH_3$ | H | $-CH_2-CH=CH-CH_2-(4)$ | - | - | H | 0,22 | |
| 44 | $-CH_3$ | $-CH_3$ | H | $-CH_2-CH=CH-CH_2-(4)$ | - | - | 2,4-Dichlor | 0,065 | |

Die bereits erwähnte synergistische Wirkung der Verbindungen der Formel I und ihrer pharmazeutisch annehmbaren Säureadditionssalze in Kombination mit anderen Sterol-Biosynthese-Hemmern, wie Ketoconazol und Terbinafin, kann beispielsweise mittels der Agar-Verdünnungs-Methode gezeigt werden. Man verwendet hierfür Casitonagar und Inokula (10 Zellen/ml) von Kulturen von Candida albicans, welche 48 Stunden alt sind. Die Testsubstanzen (TS, Verbindungen der Formel I) werden in Konzentrationen von 80-1,25 µg/ml und die Sterol-Biosynthese-Hemmer (SBH) in Konzentrationen von 20-0,001 µg/ml appliziert, wobei die Verdünnungsschritte jeweils 1:2 betragen. Die Kulturen werden jeweils während 2 Tagen bei 37°C inkubiert. Man ermittelt dann die minimalen Hemmkonzentrationen (MIC) der verschiedenen Wirkstoffe bei der alleinigen und bei der kombinierten Applikation und berechnet aus den ermittelten MIC-Werten die fraktionierte Hemmkonzentration (FIC) nach folgender Formel:

$$FIC = \frac{\text{MIC (TS allein)}}{\text{MIC (TS in Kombination)}} + \frac{\text{MIC (SBH allein)}}{\text{MIC (SBH in Kombination)}}$$

Eine synergistische Wirkung liegt vor, wenn die FIC <0,5 ist. Die in der nachfolgenden Tabelle II enthaltenen Daten für die Verbindung 6 gemäss Tabelle I, einem repräsentativen Vertreter der durch die Formel I definierten Verbindungsklasse, in Kombination mit Ketoconazol bzw. Terbinafin, repräsentativen Sterol-Biosynthese-Hemmern, belegen die synergistische Wirkung.

Tabelle II

| C.albicans | MIC in µg/ml | | | | |
|---|---|---|---|---|---|
| | Verbindung 6 allein | Ketoconazol allein | Verbindung 6 in Kombination | Ketoconazol in Kombination | FIC |
| $H_{12}$ | 40 | 5 | 1,25 | 0,155 | 0,062 |
| $H_{29}$ | 40 | 0,25 | 10 | 0,03 | 0,375 |
| $H_{42}$ | 40 | 10 | 0,6 | 0,155 | 0,032 |
| $B_5$ | 40 | 5 | 10 | 0,31 | 0,125 |
| $B_4$ | 40 | 5 | 1,25 | 0,155 | 0,062 |

| C.albicans | MIC in µg/ml | | | | |
|---|---|---|---|---|---|
| | Verbindung 6 allein | Terbinafin allein | Verbindung 6 in Kombination | Terbinafin in Kombination | FIC |
| $H_{12}$ | 40 | 100 | 2,5 | 6,25 | 0,125 |
| $H_{29}$ | 40 | 6,25 | 10 | 1,55 | 0,5 |
| $H_{42}$ | 40 | 100 | 2,5 | 6,25 | 0,125 |
| $B_5$ | 40 | 6,25 | 5 | 0,75 | 0,25 |
| $B_4$ | 40 | 12,5 | 5 | 0,55 | 0,25 |

Für die Kombination mit Verbindungen der Formel I geeignete Sterol-Biosynthese-Hemmer sind beispielsweise die systemische antifungal wirksamen Azole vom Typ des Miconazols, z.B. Ketoconazol, Itraconazol und Fluconazol, und die systemische antifungal wirksamen Allylamine vom Typ des Naftifins, z.B. Naftifin und Terbinafin.

Die Verbindungen der Formel I und ihre pharmazeutisch annehmbaren Säureadditionssalze können als Heilmittel, z.B. in Form pharmazeutischer Präparate zur enteralen, parenteralen oder topischen Applikation, Verwendung finden. Sie können beispielsweise peroral, z.B. in Form von Tabletten, Lacktabletten, Dragées, Hart- und Weichgelatinekapseln, Lösungen, Emulsionen oder Suspensionen, rektal, z.B. in Form von Suppositorien, parenteral, z.B. in Form von Injektionslösungen oder Infusionslösungen, oder topisch, z.B. in Form von Salben, Crèmen oder Oelen, verabreicht werden.

Die Herstellung der pharmazeutischen Präparate kann in jedem Fachmann geläufiger Weise dadurch erfolgen, dass man die beschriebenen Verbindungen der Formel I und ihre pharmazeutisch annehmbaren Säureadditionssalze, gegebenenfalls in Kombination mit anderen therapeutisch wertvollen Stoffen, beispielsweise den erwähnten Sterol-Biosynthese-Hemmern, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole. Für topische Präparate eignen sich als Trägermaterialien Glyceride, halbsynthetische und synthetische Glyceride, hydrierte Oele, flüssige Wachse, flüssige Paraffine, flüssige Fettalkohole, Sterole. Polyäthylenglycole und Cellulosederivate.

Als pharmazeutische Hilfsstoffe kommen die üblichen Stabilisierungs-, Konservierungs-, Netz- und Emulgiermittel, Mittel zur Verbesserung der Konsistenz, Mittel zur geschmacklichen Verbesserung, Salze zur Veränderung des osmotischen Druckes, Puffersubstanzen, Lösungsvermittler, Farbe- und Ueberzugsmittel und Antioxidantien in Frage.

Die Dosierung der Verbindungen der Formel I kann, abhängig von den zu bekämpfenden pathogenen Pilzen, dem Alter und dem individuellen Zustand des Patienten und von der Applikationsweise, innerhalb weiter Grenzen variieren und ist natürlich in jedem einzelnen Fall den individuellen Gegebenheiten anzupassen. Zur Verhütung und Bekämpfung von topischen und systemischen Infektionen durch pathogene Pilze kommt für den erwachsenen Patienten im Falle der Monotherapie eine tägliche Dosis von etwa 0,01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen. Im Falle der Kombinationstherapie kommt eine tägliche Dosis von etwa 0,01 g bis etwa 2 g, insbesondere etwa 0,02 bis etwa 1 g einer Verbindung der Formel I und von etwa 0,02 g bis etwa 0,2 g eines Sterol-Biosynthese-Hemmers in Betracht.

Die pharmazeutischen Monopräparate enthalten zweckmässigerweise etwa 10-1000 mg, vorzugsweise 50-500 mg einer Verbindung der Formel I. Die Kombinationspräparate enthalten zweckmässigerweise etwa 10-500 mg, vorzugsweise 20-250 mg einer Verbindung der Formel I und etwa 50-100 mg eines Sterol-Biosynthese-Hemmers.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

<u>Beispiel 1</u>

a) Ein Gemisch aus 2,5 g N,N-Dimethyl-6-amino-1-hexanol (Bull. Soc. Chim. France <u>1975</u>, 2315), 3,4 g 4-Hydroxybenzophenon (Beilstein 8 (III), 1263), 4,5 g Triphenylphosphin und 140 ml Tetrahydrofuran wird bei 20° langsam mit einer Lösung von 2,7 ml Azodicarbonsäure-diäthylester in 15 ml Tetrahydrofuran versetzt. Nach dem Zutropfen wird noch für 1 Stunde bei Raumtemperatur gerührt. Das Reaktionsgemisch wird im Rotationsverdampfer eingedampft, und der Rückstand wird an 400 g Aluminiumoxid neutral (Aktivitätsstufe III) mit Hexan/Essigester 7:3 chromatographiert. Das erhaltene gelbliche Oel wird in 25 ml Aether gelöst, worauf man mit 25 ml einer 10-proz. Lösung von Chlorwasserstoff in Aether versetzt. Die ausgefallenen farblosen Kristalle werden abgenutscht, mit Aether gewaschen und aus Aceton umkristallisiert.

Man erhält 1,93 g (31%) 4-[(6-Dimethylamino)hexyl)oxy]benzophenon-Hydrochlorid vom Smp. 121°.

In analoger Weise erhält man:

b) aus N,N-Dimethyl-6-amino-1-hexanol und 4'-Hydroxychalcon (Planta Med. 53, 110 (1987)) das (E)-4'-[[6-(Dimethylamino)hexyl]oxy]-3-phenylacrylophenon als farblosen Festkörper vom Smp. 43-46° (Ausbeute 27%);

c) aus N,N-Dimethyl-6-amino-1-hexanol und 4'-Hydroxy-3-phenylpropiophenon das 4'-[[6-(Dimethylamino)hexyl]oxy]-3-phenylpropiophenon als leicht gelblichen Festkörper vom Smp. 27-28° (Ausbeute 47%);

d) aus N,N-Dimethyl-6-amino-1-hexanol und 4-Fluor-4'-hydroxybenzophenon (Europäische Patentpublikationen Nos. 167240 und 128692) das 4-[[6-(Dimethylamino)hexyl]oxy]-4'-fluorbenzophenon als farblosen Festkörper vom Smp. 37-38° (Ausbeute 52%);

e) aus N,N-Dimethyl-6-amino-1-hexanol und 2-(4-Hydroxyphenyl)acetophenon (Indian J. Pharmacol. 31, 49 (1969)) das 2-[4-[[6-(Dimethylamino)hexyl]oxy]phenyl]acetophenon vom Smp. 53-54° (Ausbeute 30%);

f) aus 6-(Diäthylamino)-1-hexanol (J. Chem. Soc. 1942, 428) und 4-Hydroxybenzophenon das 4'-[[6-(Diäthylamino)hexyl]oxy]benzophenon als gelbliches Oel (Ausbeute 30%).

Massenspektrum: Spitzen u.a. bei m/e 353 ($M^+$,2%), 338 (2,7%), 199 (3%) und 86 (100%);

g) aus 6-(Diäthylamino)-1-hexanol und 3'-Hydroxychalcon (Berichte 32, 1924 (1899)) das (E)-3'-[[6-(Dimethylamino)hexyl]oxy]-3-phenylacrylophenon als gelbes Oel (Ausbeute 38%).

Massenspektrum: Spitzen u.a. bei m/e 351 ($M^+$, 8%), 131 (4%), 103 (5%) und 58 (100%);

h) aus 1-Oxo-3-phenyl-1-[4-hydroxyphenyl]propan (Chem. Zentralblatt II, 1949 (1927)) und 6-(Dimethylamino)-1-hexanol das 3'-[[6-(Dimethylamino)hexyl]oxy]-3-phenylpropiophenon als gelbliches Oel (Ausbeute 42%).

Massenspektrum: Spitzen u.a. bei m/e 353 ($M^+$, 0,8%), 128 (5,7%), 105 (1,8%) und 58 (100%);

i) aus 4-Hydroxy-2-phenylacetophenon (J. Org. Chem. 45, 1596 (1980)) und N,N-Dimethyl-6-amino-1-hexanol das 4-[(6-Dimethylamino)hexyl)oxy]-2-phenylacetophenon als farblosen Festkörper vom Smp. 69-71° (Ausbeute 25%);

j) aus 3-Hydroxybenzophenon (Beilstein 8 (III), 1262) und N,N-Dimethyl-6-amino-1-hexanol das 3-[(6-(Dimethylamino)hexyl)oxy]benzophenon als gelbliches Oel (Ausbeute 73%).

Massenspektrum: Spitzen u.a. bei m/e 325 ($M^+$, 3%), 128 (6%), 105 (6%) und 58 (100%).

Beispiel 2

a) Zu einer Lösung von 34,5 g 1,5-Dibrompentan, 9,9 g 4-Hydroxybenzophenon und 1,6 g Tetrabutylammoniumbromid in 100 ml Methylenchlorid gibt man 100 ml einer 10-proz. wässrigen Natronlauge. Das heterogene Gemisch wird über Nacht bei Raumtemperatur gerührt. Die organische Phase wird abgetrennt, über Natriumsulfat getrocknet und eingedampft. Durch Chromatographieren des Rückstandes an Kieselgel mit Hexan/Essigester 7:3 erhält man 13,47 g (78%) 4-[(5-Brompentyl)oxy]benzophenon als farbloses Oel.

Eine Lösung von 3,0 g 4-[(5-Brompentyl)oxy]benzophenon in 30 ml Aethanol wird mit 16 ml einer 33-proz. Lösung von Dimethylamin in Aethanol für 1,5 Stunden in einem Druckrohr auf 90° erhitzt. Nach dem Abkühlen wird das Gemisch auf Wasser gegossen und dreimal mit Essigester extrahiert. Die über Natriumsulfat getrockneten organischen Phasen werden eingedampft, und der Rückstand wird mit Hexan/Essigester (7:3) an Aluminiumoxid neutral (Aktivitätsstufe III) chromatographiert. Man erhält 2,69 g (97%) 4-[(5-(Dimethylamino)pentyl)oxy]benzophenon als farbloses Oel.

$^1$H-NMR ($CDCl_3$): 1-6-2,0 (m,6H); 2,23 (s,6H); 2,30 (t,J=7Hz,2H); 4,05 (t,J=7Hz,2H); 6,95 (d,J=9Hz,2H); 7,3-7,9 (m,4H); 7,83 (d,J=9Hz,2H) ppm.

In analoger Weise erhält man:

b) aus 1,8-Dibromoctan und 4-Hydroxybenzophenon das 4-[(8-Bromoctyl)oxy]benzophenon als farblose Kristalle vom Smp. 59-61° (Ausbeute 81%) und daraus mit Dimethylamin das 4-[(8-(Dimethylamino)octyl)oxy]benzophenon als gelbliches Oel (Ausbeute 42%).

$^1$H-NMR ($CDCl_3$): 1,3-1,6 (m,10H); 1,80 (qui,J=7Hz,2H); 2,22 (s,6H); 2,25 (t,J=7Hz,2H); 4,03 (t,J=7Hz,2H); 6,94 (d,J=9Hz,2H); 7,4-7,65 (m,3H); 7,7-7,9 (m,4H) ppm;

c) aus 1,6-Dibromhexan und 4-Hydroxybenzophenon das 4-[(6-Bromhexyl)oxy]benzophenon als farblose Kristalle vom Smp. 47-49° (Ausbeute 77%) und daraus mit Pyrrolidin das 4-[(6-(Pyrrolidino)hexyl)oxy]benzophenon als farbloses Oel (Ausbeute 33%).

$^1$H-NMR ($CDCl_3$): 1,3-2,0 (m,12H); 2,3-2,65 (m,6H); 4,05 (t,J=7Hz,2H); 6,97 (d,J=9Hz,2H); 7,45-8,0 (m,7H) ppm;

d) aus 1,4-Dibrombutan und 4-Hydroxybenzophenon das 4-[(4-Brombutyl)oxy]benzophenon (Ausbeute 86%) und daraus mit Dimethylamin das 4-[[4-(Dimethylamino)butyl]oxy]benzophenon als gelbliches Oel

(Ausbeute 80%).
Massenspektrum: Spitzen u.a. bei m/e 297 ($M^+$, 0,5%), 192 (0,5%), 152 (2,5%), 105 (3,5%) und 58 (100%);
e) aus 1,6-Dibromhexan und 4-Hydroxybenzophenon das 4-[(6-Bromhexyl)oxy]benzophenon (Ausbeute 66%) und daraus mit N,N-Dipropylamin das 4-[[6-(Dipropylamino)hexyl]oxy]benzophenon als gelbliches Oel (Ausbeute 94%).
Massenspektrum: Spitzen u.a. bei m/e 381 ($M^+$; 3%), 352 (60%) und 114 (100%);
f) aus 4-[(6-Bromhexyl)oxy]benzophenon und Trimethylenimin das 4-[(6-(Azetidinyl)hexyl)oxy]benzophenon als gelbliches Oel (Ausbeute 5%);
Massenspektrum: Spitzen u.a. bei m/e 337 ($M^+$; 1%), 140 (19%) und 70 (100%).
g) aus 1,7-Dibromheptan und 4-Hydroxybenzophenon das 4-[(7-Bromheptyl)oxy]benzophenon (Ausbeute 82%) und daraus mit Dimethylamin das 4-[[7-(Dimethylamino)heptyl]oxy]benzophenon als gelbliches Oel (Ausbeute 90%).
Massenspektrum: Spitzen u.a. bei m/e 339 ($M^+$, 3%), 121 (2%), 105 (3%) und 58 (100%);
h) aus 1,9-Dibromnonan und 4-Hydroxybenzophenon das 4-[(9-Bromnonyl)oxy]benzophenon (Ausbeute 74%) und daraus mit Dimethylamin das 4-[[9-(Dimethylamino)nonyl]oxy]benzophenon als farblosen Festkörper mit einem Smp. von 44-45° (Ausbeute 77%);
i) aus 1,10-Dibromdecan und 4-Hydroxybenzophenon das 4-[(10-Bromdecyl)oxy]benzophenon (Ausbeute 75%) und daraus mit Dimethylamin das 4-[[10-(Dimethylamino)decyl]oxy]benzophenon vom Smp. 33-35° (Ausbeute 90%);
j) aus 4-[(6-Bromhexyl)oxy]benzophenon und Methylamin das 4-[[6-(Methylamino)hexyl]oxy]benzophenon. Durch Behandeln mit ätherischer Salzsäure wird das entsprechende Hydrochlorid in einer Ausbeute von 13% erhalten; Smp. 155-157°;
k) aus 4-[(6-Bromhexyl)oxy]benzophenon und N-Allyl-methylamin das 4-[[6-(Allylmethylamino)hexyl]oxy]benzophenon als gelbliches Oel (Ausbeute 55%).
Massenspektrum: Spitzen u.a. bei m/e 351 ($M^+$, 5%), 84 (100%);
l) aus 1,6-Dibromhexan und 4-Hydroxy-2-phenylacetophenon das 4-[(6-Bromhexyl)oxy]-2-phenylacetophenon als gelblichen Festkörper mit einem Smp. von 75-78° (Ausbeute 71%) und daraus mit N-Allyl-methylamin das 4-[[6-(Allylmethylamino)hexyl]oxy]-2-phenylacetophenon als gelbliches Oel (Ausbeute 48%).
Massenspektrum: Spitzen u.a. bei m/e 365 ($M^+$, 4%), 274 (4%), 84 (100%);
m) aus 4-Fluor-4'-hydroxybenzophenon (Europäische Patentpublikation Nrs. 167240 und 128692) und 1,6-Dibromhexan das 4-[(6-Bromhexyl)oxy]-4'-fluor-benzophenon als farblose Kristalle mit einem Smp. von 79° (Ausbeute 57%) und daraus mit N-Allyl-methylamin das 4-[[6-(Allylmethylamino)hexyl]oxy]-4'-fluorbenzophenon, welches in das Hydrochlorid übergeführt wird (Ausbeute 74%), Smp. 84°;
n) aus trans-1,4-Dibrombuten und 4-Hydroxybenzophenon das 4-[(4-Brom-2-butenyl)oxy]benzophenon als farblosen Festkörper mit einem Smp. von 100° (Ausbeute 45%) und daraus mit Dimethylamin das trans-4-[[4-(Dimethylamino)-2-butenyl]oxy]benzophenon als farblosen Festkörper mit einem Smp. von 48-50° (Ausbeute 69%);
o) aus 4-[(4-Brom-2-butenyl)oxy]benzophenon und N-Allylmethylamin das trans-4-[[4-(Allylmethylamino)-2-butenyl]oxy]benzophenon, welches in das Hydrochlorid übergeführt wird (Ausbeute 88%); Smp. 90-91°.

## Beispiel 3

a) Ein Gemisch aus 2,17 g 6-Diäthylamino-1-hexanol (J. Chem. Soc. 1942, 428), 1,52 g 4-Hydroxybenzaldehyd und 3,3 g Triphenylphosphin in 100 ml Tetrahydrofuran wird bei 20° langsam mit einer Lösung von 1,97 g Azodicarbonsäurediäthylester versetzt. Nach dem Zutropfen wird noch 5 Stunden bei Raumtemperatur gerührt. Das Reaktionsgemisch wird dann im Rotationsverdampfer eingedampft. Der Rückstand wird an 400 g Aluminiumoxid neutral (Aktivitätsstufe III) mit Hexan/Essigester 7:3 chromatographiert. Man erhält 2,22 g (64%) 4-[[6-(Diäthylamino)hexyl]oxy]benzaldehyd als gelbliches Oel.
b) Eine Lösung dieses Aldehyds in 20 ml Aether wird zu einer Lösung von Phenäthylmagnesiumbromid (aus 1,47 g Phenäthylbromid und 143 mg Magnesium in 20 ml Aether) bei 0° unter Argon zugetropft. Das Reaktionsgemisch wird bei Raumtemperatur für 3 Stunden gerührt, dann auf 100 ml gesättigte Ammoniumchloridlösung gegossen und dreimal mit je 100 ml Dichlormethan extrahiert. Die organischen Phasen werden über Magnesiumsulfat getrocknet und eingedampft. Das rohe 1-[4-[[6-(Diäthylamino)hexyl]oxy]phenyl]-3-phenylpropanol wird ohne weitere Reinigung in die nächste Stufe eingesetzt.
c) Zu einer Lösung von 0,8 ml Oxalylchlorid in 30 ml Methylenchlorid tropft man bei -60° während 5 Minuten eine Lösung von 1,58 ml Dimethylsulfoxid in 10 ml Methylenchlorid dazu. Nach 2 Minuten Rühren gibt man bei -60° eine Lösung des rohen 1-[4-[[6-(Diäthylamino)hexyl]oxy]phenyl]-3-phenylpropanols in 20 ml Methylenchlorid innerhalb von 10 Minuten dazu. Nach 15 Minuten gibt man 7,5 ml Triäthylamin bei -60° dazu und lässt das Reaktionsgemisch auf Raumtemperatur erwärmen. Nach einer Stunde bei Raumtemperatur, gibt man 100 ml Wasser dazu, trennt die organische Phase ab und extrahiert die wässrige Phase zweimal mit je 100 ml Methylenchlorid. Man trocknet die vereinigten organischen Phasen über Magnesiumsulfat und dampft sie ein. Nach Chromatographieren des Rückstandes an 240 g Kieselgel mit Ammoniumhydroxid/Methanol/Methylenchlorid 1:10:90 erhält man 1,76 g (37%) 4'-[[6-(Diäthylamino)hexyl]oxy]-3-phenylpropiophenon als gelbes Oel, das beim Stehen kristallisiert; Smp. 83-84°.

In analoger Weise erhält man:

d) aus 3-[[6-(Diäthylamino)hexyl]oxy]benzaldehyd (hergestellt aus 3-Hydroxybenzaldehyd und 6-(Diäthylamino)-1-hexanol) und Phenäthylmagnesiumbromid und Oxidation des Kondensationsproduktes mit Oxalylchlorid/Dimethylsulfoxid das 3'-[[6-(Diäthylamino)hexyl]oxy]-3-phenylpropiophenon als gelbes Oel (Ausbeute 38%).

Massenspektrum: Spitzen u.a. bei m/e 381 ($M^+$, 1%), 366 (4%), 290 (0,8%) und 86 (100%);

e) aus 3-[[6-(Diäthylamino)hexyl]oxy]benzaldehyd und Lithiumphenylacetylid den 3-[[6-(Diäthylamino)hexyl]oxy]-α-(phenyläthinyl)benzylalkohol und daraus das 3'-[[6-(Diäthylamino)hexyl]oxy]-3-phenylpropiolophenon (Ausbeute 85%).

Massenspektrum: Spitzen u.a. bei m/e 377 ($M^+$, 1,5%), 362 (2%), 318 (3,8%), 129 (4%) und 86 (100%).

Beispiel 4

a) Zu einer Lösung von 5,52 g Pentafluorbenzol in 50 ml Tetrahydrofuran bei -78° unter Argon tropft man 20,75 ml einer 1,6M Lösung von n-Butyllithium in Hexan. Das Reaktionsgemisch wird während 30 Minuten bei -78° gerührt. Bei der gleichen Temperatur tropft man dann eine Lösung von 4,47 g Anisaldehyd in 25 ml Tetrahydrofuran dazu. Nach 1 Stunde bei -78° lässt man das Gemisch auf Raumtemperatur erwärmen und rührt noch während 1 Stunde weiter. Das Reaktionsgemisch wird auf 100 ml einer gesättigten Ammoniumchloridlösung gegossen und dreimal mit je 100 ml Essigester extrahiert. Man wäscht die organischen Phasen mit 100 ml einer gesättigten Natriumchloridlösung und trocknet sie über Magnesiumsulfat. Nach Eindampfen erhält man 10 g rohes 2,3,4,5,6-Pentafluorphenyl-4'-methoxyphenylcarbinol.

b) Eine Lösung dieses rohen Carbinols in 60 ml Methylenchlorid wird zu einer Lösung von 3,3 ml Oxalylchlorid und 5,6 ml Dimethylsulfoxid in 130 ml Methylenchlorid innerhalb von 15 Minuten bei -78° unter Argon dazugetropft. Das Gemisch wird während 15 Minuten bei -78° gerührt. 27,6 ml Triäthylamin werden dann bei -78° innerhalb von 15 Minuten dazugetropft. Man lässt dann das Gemisch auf Raumtemperatur erwärmen. Das Reaktionsgemisch wird mit 500 ml Wasser versetzt. Die organische Phase wird abgetrennt, und die wässrige Phase wird zweimal mit je 200 ml Methylenchlorid extrahiert. Die vereinigten organischen Phasen werden über Magnesiumsulfat getrocknet und im Rotationsverdampfer eingedampft. Der Rückstand wird an 920 g Kieselgel mit Methylenchlorid chromatographiert. Man erhält 7,82 g (78%) 2,3,4,5,6-Pentafluor-4'-methoxy-benzophenon als farbloses Oel.

c) Eine Lösung von 5,6 g 2,3,4,5,6-Pentafluor-4'-methoxybenzophenon in 100 ml Aethylenchlorid wird zu einer Lösung von 23,23 g Bortribromid in 100 ml Aethylenchlorid bei -7° unter Argon dazugetropft. Das Reaktionsgemisch wird auf Raumtemperatur erwärmt und während 5 Stunden bei dieser Temperatur gerührt. Man giesst das Reaktionsgemisch auf 200 ml eiskaltes Wasser und extrahiert dreimal mit je 150 ml Methylenchlorid. Die vereinigten Extrakte werden mit 200 ml Wasser gewaschen und über Magnesiumsulfat getrocknet. Nach Abdampfen des Lösungsmittels chromatographiert man den Rückstand an 900 g Kieselgel mit Methylenchlorid. Man erhält 3,87 g (72%) 2,3,4,5,6-Pentafluor-4'-hydroxybenzophenon mit einem Smp. von 138-140°.

d) In Analogie zu Beispiel 1 erhält man aus 2,3,4,5,6-Pentafluor-4'-hydroxybenzophenon und 6-(Dimethylamino)-1-hexanol das 4'-[[6-(Dimethylamino)hexyl]oxy]-2,3,4,5,6-Pentafluorbenzophenon als farbloses Oel (Ausbeute 37%).

$^1H$-NMR ($CDCl_3$): 1,3-1,9 (m,8H); 2,26 (s,6H); 2,1-2,3 (m,2H); 4,06 (t,J=7Hz,2H); 6,96 (d,J=9Hz,2H); 7,84 (d,J=9Hz,2H) ppm.

Beispiel 5

a) In Analogie zu Beispiel 3b) erhält man aus 4-[[6-(Diäthylamino)hexyl]oxy]benzaldehyd und Phenylacetylen/Butyllithium rohes 4'-[[6-(Diäthylamino)hexyl]oxy]phenyl-phenyläthinyl-carbinol (Ausbeute 50%).

b) Eine Lösung von 1,58 g 4'-[[6-(Diäthylamino)hexyl]oxy]phenyl-phenyläthinyl-carbinol in 60 ml Methylenchlorid wird mit 2,07 g aktiviertem Mangandioxid versetzt. Nach 20 Stunden wird das Reaktionsgemisch durch Kieselgur filtriert. Nach Eindampfen des Filtrats wird der Rückstand an 100 g Aluminiumoxyd neutral

(Aktivitätsstufe III) mit Hexan/Essigester 2:1 chromatographiert. Man erhält 1,34 g (50%) 4'-[[6-(Diäthylamino)hexyl]oxy]-3-phenylpropiolophenon als farbloses Oel.
Massenspektrum: Spitzen u.a. bei m/e 377 ($M^+$, 5%), 362 (4,8%), 194 (3%) und 86 (100%).

Beispiel 6

a) Eine Lösung von 24 g 2-Methylanisol, 35 g N-Bromsuccinimid und 1,24 g Dibenzoyl-peroxid in 150 ml Tetrachlorkohlenstoff wird während 12 Stunden unter Rückfluss zum Sieden erhitzt. Nach Erkalten der Lösung wird filtriert, und das Filtrat wird eingedampft. Der Rückstand wird aus Hexan umkristallisiert. Man erhält 18,6 g (47%) 4-Brom-2-methylanisol als farblosen Festkörper mit einem Smp. von 66-68°.
b) Aus 10 g 4-Brom-2-methylanisol und 1,21 g Magnesiumspänen in 30 ml Tetrahydrofuran wird das entsprechende Grignard-Reagens hergestellt, welches tropfenweise in eine auf -72° vorgekühlte Lösung von 7 g Benzoylchlorid in 80 ml Tetrahydrofuran gegeben wird. Die Temperatur wird dabei unterhalb -65° gehalten. Nach dem Zutropfen wird noch während 0,5 Stunden bei 20° gerührt, worauf man mit 400 ml Eiswasser hydrolysiert. Die mit verdünnter Salzsäure angesäuerte Lösung wird mit Aether extrahiert, und die organische Phase wird getrocknet und eingedampft. Durch Chromatographieren des Rückstandes an Kieselgel mit Hexan/Essigester 7:3 erhält man 6,17 g (55%) 4-Methoxy-3-methyl-benzophenon als gelbliches Oel.
$^1$H-NMR ($CDCl_3$): 2,26 (s,3H); 3,90 (s,3H); 6,8-8,0 (m,8H) ppm.
c) Ein Gemisch von 6,17 g 4-Methoxy-3-methyl-benzophenon, 65 ml Eisessig und 103 ml einer 62-proz. Bromwasserstoffsäurelösung wird während 3 Stunden unter Rückfluss zum Sieden erhitzt. Die erhaltene dunkle Lösung wird eingeengt, mit Essigester extrahiert, und die organische Phase wird getrocknet und eingedampft. Der Rückstand wird mit Hexan/Essigester 7:3 an Kieselgel chromatographiert. Man erhält 2,90 g (50%) 3-Methyl-4-hydroxybenzophenon als gelblichen Festkörper mit einem Smp. von 170-173°.
d) In Analogie zu Beispiel 2 erhält man aus 1,6-Dibromhexan und 3-Methyl-4-hydroxybenzophenon das 4-[(6-Bromhexyl)oxy-3-methyl-benzophenon als gelbliches Oel [Ausbeute 79%; $^1$H-NMR ($CDCl_3$): 1,45-2,15 (m,8H); 2,25 (s,3H); 3,43 (t,J=7Hz,2H); 4,07 (t,J=7Hz,2H); 6,8-8,0 (m,8H) ppm] und daraus mit Dimethylamin das 4-[[6-(Dimethylamino)hexyl]oxy]-3-methyl-benzophenon, welches in das Hydrochlorid übergeführt wird (Ausbeute 61%); Smp. 162-163°.

Beispiel 7

In Analogie zu Beispiel 2 erhält man aus 4-[(6-Bromhexyl)oxy]-3-methyl-benzophenon und N-Allyl-methylamin das 4-[[6-(Allylmethylamino)hexyl]oxy]-3-methyl-benzophenon, welches in das Hydrochlorid übergeführt wird (Ausbeute 49%); Smp. 112-113°.

Beispiel 8

a) Aus dem Grignard-Reagens von 2-Brom-m-xylol und 4-Methoxybenzoylchlorid erhält man in Analogie zu Beispiel 6b) das 4-Methoxy-2',3'-dimethylbenzophenon als gelbliches Oel (Ausbeute 47%).
$^1$H-NMR ($CDCl_3$): 2,15 (s,6H); 3,85 (s,3H); 6,8-7,95 (m,7H) ppm.
b) In Analogie zu Beispiel 6c) erhält man daraus das 4-Hydroxy-2',3'-dimethylbenzophenon als gelbliche Kristalle (Ausbeute 72%); Smp. 155-158°.
c) In Analogie zu Beispiel 2 erhält man daraus mit 1,6-Dibromhexan das 4-[(6-Bromhexyl)oxy]-2',3'-dimethyl-benzophenon als gelbliches Oel [Ausbeute 78%; $^1$H-NMR ($CDCl_3$): 1,35-2,0 (m,8H); 2,11 (s,6H); 3,43 (t,J=7Hz,2H); 4,03 (t,J=7Hz,2H); 6,8-7,9 (m,7H) ppm] und daraus mit Dimethylamin das 4-[[6-(Dimethylamino)hexyl]oxy]-2',3'-dimethylbenzophenon, welches in das Hydrochlorid übergeführt wird (Ausbeute 81%); Smp. 117-120°.

Beispiel 9

Aus 4-[(6-Bromhexyl)oxy]-2',3'-dimethyl-benzophenon und N-Allyl-methylamin erhält man in Analogie zu Beispiel 2 das 4-[[6-(Allylmethylamino)hexyl]oxy]-2',3'-dimethyl-benzophenon als gelbliches Oel (Ausbeute 82%).
Massenspektrum: Spitzen u.a. bei m/e 379 ($M^+$, 4%), 350 (6%), 84 (100%).

Beispiel 10

a) Aus dem Grignard-Reagens von 4-Bromanisol und 2,4-Dichlorbenzoylchlorid erhält man in Analogie zu Beispiel 6b) das 2,4-Dichlor-4'-methoxybenzophenon als gelbes Oel (Ausbeute 76%). $^{1}$H-NMR ($CDCl_3$): 3,56 (s,3H); 6,9-8,0 (m,7H).
b) In Analogie zu Beispiel 6c) erhält man daraus das 4-Hydroxy-2',4'-dichlorbenzophenon als gelbliche Kristalle (Ausbeute 63%); Smp. 140°.
c) In Analogie zu Beispiel 2 erhält man daraus mit 1,6-Dibromhexan das 4-[(6-Bromhexyl)oxy]-2',4'-dichlorbenzophenon als farbloses Oel [Ausbeute 86%; Massenspektrum: Spitzen u.a. bei m/e 430 ($M^+$, 10%), 266 (19%), 173 (18%), 121 (100%)] und daraus mit N-Allyl-methylamin das 4-[[6-Allylmethylamino)hexyl]oxy]-2',4'-dichlorbenzophenon als gelbliches Oel (Ausbeute 67%).
Massenspektrum: Spitzen u.a. bei m/e 419 ($M^+$, 2%), 173 (3%), 84 (100%).

Beispiel 11

Aus 4-Hydroxy-2',4'-dichlorbenzophenon und N,N-Dimethyl-6-amino-1-hexanol erhält man in Analogie zu Beispiel 1 das 4-[[6-(Dimethylamino)hexyl]oxy]-2',4'-dichlorbenzophenon als farbloses Oel (Ausbeute 31%). Massenspektrum: Spitzen u.a. bei m/e 393 (0,5%), 173 (1,5%), 128 (5%), 58 (100%).

Beispiel 12

Aus der Grignard-Verbindung von 4-Bromanisol und 3,5-Dichlorbenzoylchlorid erhält man in Analogie zu Beispiel 6b) das 3,5-Dichlor-4'-methoxybenzophenon als farblosen Festkörper (Ausbeute 37%).
$^{1}$H-NMR ($CDCl_3$): 3,92 (s,3H); 7,0-8,0 (m,7H) ppm.

In Analogie zu Beispiel 6c) erhält man daraus das 4-Hydroxy-3',5'-dichlorbenzophenon als bräunliche Kristalle (Ausbeute 91%); Smp. 183°.

In Analogie zu Beispiel 2 erhält man daraus mit 1,6-Dibromhexan das 4-[(6-Bromhexyl)oxy]-3',5'-dichlorbenzophenon als farblosen Festkörper (Ausbeute 49%) mit einem Smp. von 70° und daraus mit Dimethylamin das 4-[[6-(Dimethylamino)hexyl]oxy]-3',5'-dichlorbenzophenon, welches in das Hydrochlorid übergeführt wird (Ausbeute 66%); Smp. 148-149°.

Beispiel 13

Aus 4-[(6-Bromhexyl)oxy]-3',5'-dichlorbenzophenon und N-Allylmethylamin erhält man in Analogie zu Beispiel 2 das 4-[[6-(Allylmethylamino)hexyl]oxy]-3',5'-dichlorbenzophenon, welches in das Hydrochlorid übergeführt wird (Ausbeute 74%); Smp. 111-112°.

Beispiel 14

Aus 4-Hydroxy-2',4'-dichlorbenzophenon und trans-1,4-Dibrombuten erhält man in Analogie zu Beispiel 2 das 4-[(4-Brom-2-butenyl)oxy]-2',4'-dichlorbenzophenon als klebriges Harz [Ausbeute 73%; Massenspektrum: Spitzen u.a. bei m/e 400 ($M^+$, 2%), 319 (3%), 266 (36%), 173 (54%), 133 (45%), 121 (70%)] und daraus mit Dimethylamin das trans-4-[[4-(Dimethylamino)-2-butenyl]oxy]-2',4'-dichlorbenzophenon, welches in das Hydrochlorid übergeführt wird (Ausbeute 82%); Smp. 147°.

Beispiel 15

Aus 4-[(4-Brom-2-butenyl)oxy]-2',4'-dichlorbenzophenon und N-Allyl-methylamin erhält man in Analogie zu Beispiel 2 das trans-4-[[4-(Allylmethylamino)-2-butenyl]oxy]-2',4'-dichlorbenzophenon, welches in das Hydrochlorid übergeführt wird (Ausbeute 80%); Smp. 98°.

Beispiel 16

a) 150 ml Nitrobenzol werden im Eisbad gekühlt und portionenweise mit 41 g Aluminiumchlorid versetzt. Die Temperatur wird dabei unterhalb 5° gehalten. Anschliessend wird eine Lösung von 50 g 4-Nitrobenzoylchlorid in 50 ml Nitrobenzol so zugetropft, dass die Temperatur unterhalb 5° bleibt. Bei der gleichen Temperatur werden nun 27,8 g Anisol dazu getropft. Anschliessend wird über Nacht bei 20° gerührt. Die Lösung wird auf 1 l Eiswasser gegossen, mit Methylenchlorid extrahiert, über Magnesiumsulfat

getrocknet und eingeengt. Das Nitrobenzol wird im Hochvakuum abdestilliert, und die verbleibenden gelblichen Kristalle werden aus Cyclohexan umkristallisiet. Man erhält 36,5 g (52%) 4-Methoxy-4'-nitrobenzophenon als gelben Festkörper mit einem Smp. von 124°.

b) Aus 4-Methoxy-4'-nitrobenzophenon und Bromwasserstoffsäure erhält man in Analogie zu Beispiel 6c) das 4-Hydroxy-4'-nitrobenzophenon als gelbe Kristalle (Ausbeute 75%); Smp. 196°.

c) In Analogie zu Beispiel 2 erhält man aus 4-Hydroxy-4'-nitrobenzophenon und 1,6-Dibromhexan das 4-[(6-Bromhexyl)oxy]-4'-nitrobenzophenon als gelben Festkörper mit einem Smp. von 61° (Ausbeute 65%) und daraus mit Dimethylamin das 4-[[6-(Dimethylamino)hexyl]oxy]-4'-nitrobenzophenon, welches in das Hydrochlorid übergeführt wird (Ausbeute 80%); Smp. 101°.

Beispiel 17

Aus 4-[(6-Bromhexyl)oxy]-4'-nitrobenzophenon und N-Allylmethylamin erhält man in Analogie zu Beispiel 2 das 4-[[6-(Allylmethylamino)hexyl]oxy]-4'-nitrobenzophenon, welches in das Hydrochlorid übergeführt wird (Ausbeute 85%); Smp. 79°.

Beispiel 18

Aus 2-Chloranisol und Benzoylchlorid erhält man in Analogie zu Beispiel 16a) das 3-Chlor-4-methoxybenzophenon als farblosen Festkörper (Ausbeute 72%), Smp. 87°.

In Analogie zu Beispiel 6c) erhält man daraus das 3-Chlor-4-hydroxybenzophenon als gelblichen Festkörper (Ausbeute 91%); Smp. 180°.

In Analogie zu Beispiel 2 erhält man daraus mit 1,6-Dibromhexan das 4-[(6-Bromhexyl)oxy]-3-chlor-benzophenon als farblosen Festkörper mit einem Smp. von 58° (Ausbeute 68%) und daraus mit Dimethylamin das 4-[[6-(Dimethylamino)hexyl]oxy]-3-chlor-benzophenon, welches in das Hydrochlorid übergeführt wird (Ausbeute 94%); Smp. 195°.

Beispiel 19

Aus 4-[(6-Bromhexyl)oxy]-3-chlor-benzophenon und N-Allyl-methylamin erhält man in Analogie zu Beispiel 2 das 4-[[6-(Allylmethylamino)hexyl]oxy]-3-chlor-benzophenon, welches in das Hydrochlorid übergeführt wird (Ausbeute 98%); Smp. 133°.

Beispiel 20

Aus 2-Chloranisol und 2,4-Dichlorbenzoylchlorid erhält man in Analogie zu Beispiel 16a) das 2',3,4'-Trichlor-4-methoxybenzophenon als gelbliche Kristalle (Ausbeute 87%); Smp. 98°.

Daraus erhält man in Analogie zu Beispiel 6c) das 2',3,4'-Trichlor-4-hydroxy-benzophenon als gelbliche Kristalle (Ausbeute 89%); Smp. 145°.

In Analogie zu Beispiel 2 erhält man daraus mit 1,6-Dibromhexan das 4-[(6-Bromhexyl)oxy]-2',3,4'-trichlorbenzophenon als gelbliches Oel (Ausbeute 21%) und daraus mit Dimethylamin das 4-[[6-(Dimethylamino)hexyl]oxy]-2',3,4'-trichlor-benzophenon, welches in das Hydrochlorid übergeführt wird (Ausbeute 49%); Smp. 91°.

Beispiel 21

Aus 4-[(6-Bromhexyl)oxy]-2',3,4'-trichlorbenzophenon und N-Allyl-methylamin erhält man in Analogie zu Beispiel 2 das 4-[[6-(Allylmethylamino)hexyl]oxy]-2',3,4'-trichlorbenzophenon, welches in das Hydrochlorid übergeführt wird (Ausbeute 38%); Smp. 100°.

Beispiel 22

In Analogie zu Beispiel 2 erhält man aus 4-Hydroxy-4'-nitrobenzophenon und trans-1,4-Dibrombuten das 4-[(4-Brom-2-butenyl)oxy]-4'-nitrobenzophenon als gelblichen Festkörper [Ausbeute 71%; Massenspektrum: Spitzen u.a. bei m/e 375 ($M^+$, 2%), 296 (8%), 243 (44%), 150 (32%), 133 (62%), 121 (62%)] und daraus mit Dimethylamin das trans-4-[[4-(Dimethylamino)-2-butenyl]oxy]-4'-nitrobenzophenon, welches in das Hydrochlorid übergeführt wird (Ausbeute 90%); Smp. 144°.

Beispiel 23

Aus 4-[(4-Brom-2-butenyl)oxy]-4'-nitrobenzophenon und N-Allyl-methylamin erhält man in Analogie zu Beispiel 2 das trans-4-[[4-(Allylmethylamino)-2-butenyl]oxy]-4'-nitrobenzophenon, welches in das Hydrochlorid übergeführt wird (Ausbeute 85%); Smp. 152°.

Beispiel 24

Aus 4-Brombenzoylchlorid und Anisol erhält man in Analogie zu Beispiel 16a) das 4-Brom-4'-methoxybenzophenon als farblosen Festkörper (Ausbeute 65%).
Massenspektrum: Spitzen u.a. bei m/e 290 ($M^+$, 18%), 135 (100%).

Daraus erhält man in Analogie zu Beispiel 6c) das 4-Brom-4'-hydroxybenzophenon als gelblichen Festkörper (Ausbeute 77%).
Massenspektrum: Spitzen u.a. bei m/e 276 ($M^+$, 18%), 121 (100%).

In Analogie zu Beispiel 2 erhält man daraus mit trans-1,4-Dibrombuten das 4-[(4-Brom-2-butenyl)oxy]-4'-brombenzophenon als farblose Kristalle [Ausbeute 64%; Massenspektrum: Spitzen u.a. bei m/e 410 ($M^+$, 4%), 329 (11%), 276 (32%), 183 (34%), 133 (50%), 121 (100%)] und daraus mit Dimethylamin das trans-4-[[4-(Dimethylamino)-2-butenyl]oxy]-4'-brombenzophenon, welches in das Hydrochlorid übergeführt wid (Ausbeute 85%); Smp. 184-185°.

Beispiel 25

Aus 4-[(4-Brom-2-butenyl)oxy]-4'-brombenzophenon und N-Allylmethylamin erhält man in Analogie zu Beispiel 2 das trans-4-[[4-(Allylmethylamino)-2-butenyl]oxy]-4'-brombenzophenon, welches in das Hydrochlorid übergeführt wird (Ausbeute 83%); Smp. 116-117°.

Beispiel A

Die Verbindung 4-[(6-(Dimethylamino)hexyl)oxy]-2-phenylacetophenon kann wie folgt als Wirkstoff zur Herstellung von Tabletten verwendet werden:

| Bestandteile | | mg/Tablette |
|---|---|---|
| Wirkstoff | | 200 |
| Milchzucker pulv. | | 100 |
| Povidone K 30 | | 15 |
| Na-Carboxymethylstärke | | 10 |
| Talk | | 3 |
| Magnesiumstearat | | 2 |
| | Tablettengewicht | 330 |

Der Wirkstoff und der Milchzucker pulv. werden intensiv gemischt. Die erhaltene Mischung wird dann mit einer wässrigen Lösung von Povidone K 30 befeuchtet und geknetet, worauf man die erhaltene Masse granuliert, trocknet und siebt. Man mischt das Granulat mit den übrigen Bestandteilen und verpresst dann zu Tabletten geeigneter Grösse.

## Patentansprüche

### Patentansprüche für folgende Vertragsstaaten : AT, BE, CH, DE, DK, FR, GB, IT, LI, LU, NL, SE

1. Verwendung von Verbindungen der allgemeinen Formel

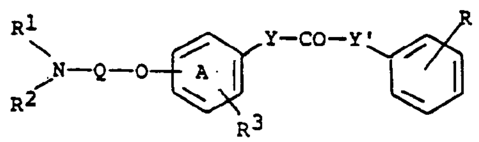

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q-O- an die 3- oder 4-Stellung des mit A bezeichneten Ringes gebunden ist und das Symbol R bedeutet, dass der Ring unsubstituiert oder durch Halogen, Trifluormethyl, Cyano, Nitro, niederes Alkyl und/oder niederes Alkoxy einfach oder mehrfach substituiert ist,
und ihren pharmazeutisch annehmbaren Säureadditionssalzen, gegebenenfalls in Kombination mit antifungal wirksamen Substanzen, welche die Sterol-Biosynthese hemmen, zur Herstellung von antifungal wirksamen Mitteln.

2. Verbindungen der allgemeinen Formel

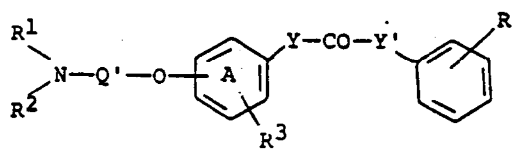

Ia

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q' Alkylen mit 5 bis 11 Kohlenstoffatomen und mindestens 5 Kohlenstoffatomen zwischen den zwei freien Valenzen oder Alkenylen mit 4 bis 11 Kohlenstoffatomen und mindestens 4 Kohlenstoffatomen zwischen den zwei Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q'-O- an die 3- oder 4-Stellung des mit A bezeichneten Ringes gebunden ist und das Symbol R bedeutet, dass der Ring unsubstituiert oder durch Trifluormethyl, Cyano, niederes Alkyl und/oder niederes Alkoxy einfach oder mehrfach substituiert ist,
und ihre pharmazeutisch annehmbaren Säureadditionssalze, zur Anwendung als therapeutische Wirkstoffe, insbesondere als antifungal wirksame Stoffe.

3. Verbindungen der allgemeinen Formel

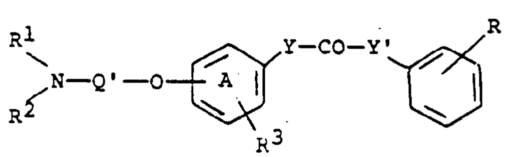

Ia

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q' Alkylen mit 5 bis 11 Kohlenstoffatomen und mindestens 5 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q'-O- an die 3- oder 4-Stellung des mit A bezeichneten Ringes gebunden ist und das Symbol R bedeutet, dass der Ring unsubstituiert oder durch Trifluormethyl, Cyano, niederes Alkyl und/oder niederes Alkoxy einfach oder mehrfach substituiert ist, mit der Massgabe, dass Y und

Y' nicht gleichzeitig eine direkte Bindung bedeuten, wenn Q' Alkylen mit 5 Kohlenstoffatomen $R^1$ und $R^2$ gleichzeitig niederes Alkyl mit mehr als 2 Kohlenstoffatomen bedeuten,
und ihre pharmazeutisch annehmbaren Säureadditionssalze.

**4.** Verbindungen nach Anspruch 2 oder 3, worin $R^1$ und $R^2$ je Wasserstoff oder niederes Alkyl oder zusammen geradkettiges Alkylen mit 2 bis 4 Kohlenstoffatomen, $R^3$ Wasserstoff und Q' Alkylen mit 5 bis 11 Kohlenstoffatomen und mindestens 5 Kohlenstoffatomen zwischen den zwei freien Valenzen bedeuten, und das Symbol R bedeutet, dass der Ring unsubstituiert oder durch, Trifluormethyl, niederes Alkyl oder niederes Alkoxy substituiert ist.

**5.** Verbindungen nach einem der Ansprüche 2 bis 4, worin Q' unverzweigtes Alkylen mit 5 bis 7 Kohlenstoffatomen bedeutet.

**6.** Verbindungen nach einem der Ansprüche 2, 3 und 5, worin $R^1$ und $R^2$ je $C_{1-4}$-Alkyl oder $C_{3-4}$-Alkenyl oder zusammen $C_{3-4}$-Alkylen bedeuten.

**7.** Verbindungen nach einem der Ansprüche 2 bis 6, worin die Gruppe $R^1R^2N$-Q'-O- an die 4-Stellung des mit A bezeichneten Ringes gebunden ist.

**8.** Verbindungen nach einem der Ansprüche 2 bis 7, worin Y eine direkte Bindung oder die Gruppe $-CH_2-$ bedeutet.

**9.** Verbindungen nach Anspruch 8, worin Y eine direkte Bindung bedeutet.

**10.** Verbindungen nach einem der Ansprüche 2 bis 9, worin Y' eine direkte Bindung oder die Gruppe $-CH_2-$, $-CH_2CH_2-$ oder $-CH{=}CH-$ bedeutet.

**11.** Verbindungen nach Anspruch 10, worin Y' eine direkte Bindung oder die Gruppe $-CH_2-$ bedeutet.

**12.** Verbindungen nach einem der Ansprüche 2 bis 11, worin das Symbol R bedeutet, dass der Ring unsubstituiert oder durch niederes Alkyl mono- oder disubstituiert ist.

**13.** 4-[(6-(Dimethylamino)hexyl)oxy]-2-phenylacetophenon,
4-[(6-(Dimethylamino)hexyl)oxy]benzophenon,
4'-[(6-(Diäthylamino)hexyl)oxy]-3-phenylpropiophenon,
4-[(6-(Dimethylamino)hexyl)oxy]-3-phenylpropiophenon,
4'-[(6-(Dimethylamino)hexyl)oxy]-3-phenylacrylophenon,
4-[(6-(Dimethylamino)hexyl)oxy]benzophenon,
4-[(6-(1-Azetidinyl)hexyl)oxy]benzophenon,
4-[(6-(1-Pyrrolidinyl)hexyl)oxy]benzophenon,
4-[4-[(6-(Dimethylamino)hexyl)oxy]phenyl]acetophenon,
4-[(7-(Dimethylamino)heptyl)oxy]benzophenon oder
4-[(5-(Dimethylamino)pentyl)oxy]benzophenon als Verbindung nach Anspruch 2, 3 oder 4.

**14.** 4-[[6-(Allylmethylamino)hexyl]oxy]-2-phenylacetophenon,
trans-4-[[4-(Allylmethylamino)-2-butenyl]oxy]benzophenon,
4-[[6-(Allylmethylamino)hexyl]oxy]-3-methylbenzophenon,
oder
4-[[6-(Allylmethylamino)hexyl]oxy]-3-chlorbenzophenon als Verbindung nach Anspruch 2 oder 3.

**15.** Verfahren zur Herstellung von Verbindungen nach einem der Ansprüche 3 bis 14 und von pharmazeutisch annehmbaren Säureadditionssalzen davon, dadurch gekennzeichnet, dass man
a) eine Verbindung der allgemeinen Formel

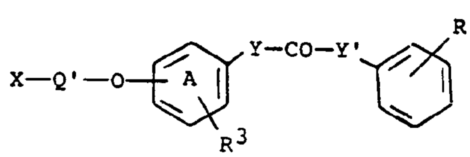

II

worin X eine Abgangsgruppe bedeutet, und A, Q', Y, Y', $R^3$ und R die in Anspruch 3 angegebene Bedeutung besitzen,
mit einem Amin der allgemeinen Formel $HNR^1R^2$, worin $R^1$ und $R^2$ die in Anspruch 3 angegebene Bedeutung besitzen, umsetzt, oder
b) eine Verbindung der allgemeinen Formel

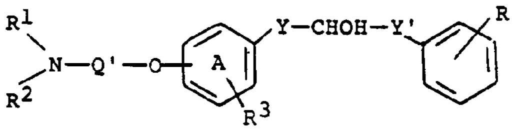

III

worin A, $R^1$, $R^2$, $R^3$, Q', Y, Y' und R die in Anspruch 3 angegebene Bedeutung besitzen,
oxidiert, oder
c) eine Verbindung der allgemeinen Formel

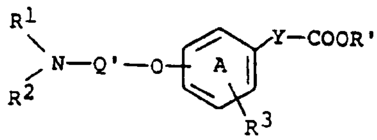

IV

worin R' niederes Alkyl bedeutet, und A, $R^1$, $R^2$, $R^3$, Q' und Y die in Anspruch 3 angegebene Bedeutung besitzen,
mit einer Verbindung der allgemeinen Formel

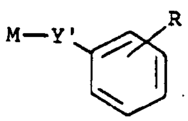

V

worin M -MgCl, -MgBr, -MgJ oder -Li bedeutet, und Y' und R die in Anspruch 3 angegebene Bedeutung besitzen,
umsetzt, oder
d) eine Verbindung der allgemeinen Formel

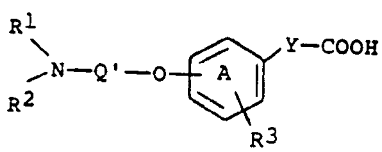

VI

worin A, $R^1$, $R^2$, $R^3$, Q' und Y die in Anspruch 3 angegebene Bedeutung besitzen,
in Form eines reaktiven Derivates in Gegenwart einer Lewis-Säure mit einer Verbindung der allgemeinen Formel

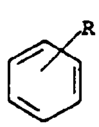

VII

worin R die in Anspruch 3 angegebene Bedeutung besitzt,
umsetzt, oder
e) eine Verbindung der allgemeinen Formel

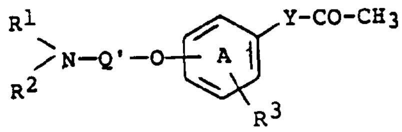

VIII

worin A, $R^1$, $R^2$, $R^3$, Q' und Y die in Anspruch 3 angegebene Bedeutung besitzen, in Gegenwart einer Base mit einer Verbindung der allgemeinen Formel

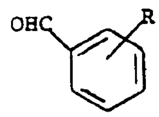

IX

worin R die in Anspruch 3 angegebene Bedeutung besitzt, umsetzt, oder

f) eine Verbindung der allgemeinen Formel

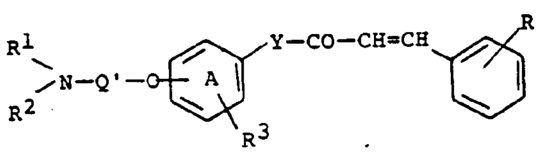

I'

worin A, $R^1$, $R^2$, $R^3$, Q', Y und R die in Anspruch 3 angegebene Bedeutung besitzen, hydriert, oder

g) eine Verbindung der allgemeinen Formel

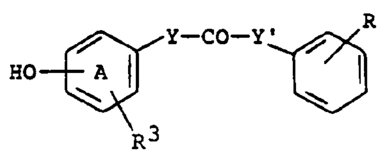

X

worin A, R, $R^3$, Y und Y' die in Anspruch 3 angegebene Bedeutung besitzen, in Gegenwart von Triphenxylphosphin und einem Azodicarbonsäure-di(niederem Alkylester) mit einer Verbindung der allgemeinen Formel

$R^1R^2N\text{-}Q'\text{-}OH$ XI

worin $R^1$, $R^2$ und Q' die in Anspruch 3 angegebene Bedeutung besitzen, umsetzt, und

h) eine erhaltene Verbindung der Formel Ia erwünschtenfalls in ein pharmazeutisch annehmbares Säureadditionssalz überführt.

**16.** Arzneimittel, enthaltend eine Verbindung gemäss einem der Ansprüche 2 bis 14 und einen therapeutisch inerten Träger.

**17.** Antifungal wirksame Mittel, enthaltend eine Verbindung gemäss einem der Ansprüche 2 bis 14 und einen therapeutisch inerten Träger.

**18.** Arzneimittel nach Anspruch 16 oder 17, dadurch gekennzeichnet, dass sie als zusätzlichen Wirkstoff eine andere antifungal wirksame Substanz, welche die Sterol-Biosynthese hemmt, enthalten.

**19.** Verwendung von Verbindungen gemäss einem der Ansprüche 2 bis 14 gegebenenfalls in Kombination mit anderen antifungal wirksamen Substanzen, welche die Sterol-Biosynthese hemmen, für die Herstellung von antifungal wirksamen Mitteln.

**Patentansprüche für folgende Vertragsstaaten : ES, GR**

1. Verwendung von Verbindungen der allgemeinen Formel

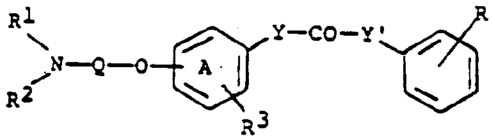

I

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q Alkylen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q-O- an die 3- oder 4-Stellung des mit A bezeichneten Ringes gebunden ist und das Symbol R bedeutet, dass der Ring unsubstituiert oder durch Halogen, Trifluormethyl, Cyano, Nitro, niederes Alkyl und/oder niederes Alkoxy einfach oder mehrfach substituiert ist,
und ihren pharmazeutisch annehmbaren Säureadditionssalzen, gegebenenfalls in Kombination mit antifungal wirksamen Substanzen, welche die Sterol-Biosynthese hemmen, zur Herstellung von antifungal wirksamen Mitteln.

2. Verwendung von Verbindungen der allgemeinen Formel

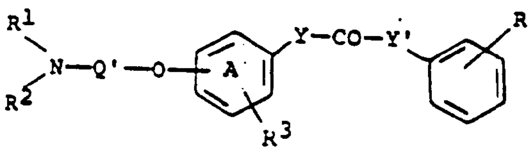

Ia

worin $R^1$ und $R^2$ je Wasserstoff, niederes Alkyl oder niederes Alkenyl oder zusammen geradkettiges Alkylen mit 2 bis 4 Kohlenstoffatomen, $R^3$ Wasserstoff, Halogen oder niederes Alkyl, Q' Alkylen mit 5 bis 11 Kohlenstoffatomen und mindestens 5 Kohlenstoffatomen zwischen den zwei freien Valenzen oder Alkenylen mit 4 bis 11 Kohlenstoffatomen und mindestens 4 Kohlenstoffatomen zwischen den zwei Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q'-O- an die 3- oder 4-Stellung des mit A bezeichneten Ringes gebunden ist und das Symbol R bedeutet, dass der Ring unsubstituiert oder durch Halogen, Trifluormethyl, Cyano, Nitro, niederes Alkyl und/oder niederes Alkoxy einfach oder mehrfach substituiert ist,
und ihren pharmazeutisch annehmbaren Säureadditionssalzen, gegebenenfalls in Kombination mit antifungal wirksamen Substanzen, welche die Sterol-Biosynthese hemmen, zur Herstellung von antifungal wirksamen Mitteln.

3. Verfahren zur Herstellung von Verbindungen der allgemeinen Formel

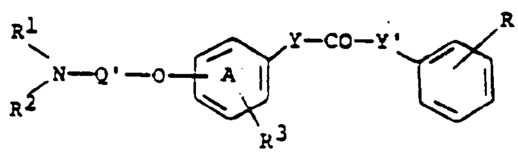

Ia

worin $R^1$ und $R^2$ je Wasserstoff. niederes Alkyl oder niederes Alkenyl oder zusammen geradkettiges Alkylen mit 2 bis 4 Kohlenstoffatomen, $R^3$ Wasserstoff, Halogen oder niederes Alkyl, Q' Alkylen mit 5 bis 11 Kohlenstoffatomen und mindestens 5 Kohlenstoffatomen zwischen den zwei freien Valenzen oder Alkenylen mit 4 bis 11 Kohlenstoffatomen und mindestens 4 Kohlenstoffatomen zwischen den zwei freien Valenzen und Y und Y' je eine direkte Bindung oder die Gruppe $-CH_2-$, $-CH_2CH_2-$, -CH=CH- oder -C≡C- bedeuten, die Gruppe $R^1R^2N$-Q'-O- an die 3- oder 4-Stellung des mit A bezeichneten Ringes gebunden ist und das Symbol R bedeutet, dass der Ring unsubstituiert oder durch Trifluormethyl, Cyano, niederes Alkyl und/oder niederes Alkoxy einfach oder mehrfach substituiert ist, mit der Massgabe, dass Y und

Y' nicht gleichzeitig eine direkte Bindung bedeuten, wenn Q' Alkylen mit 5 Kohlenstoffatomen und $R^1$ und $R^2$ gleichzeitig niederes Alkyl mit mehr als 2 Kohlenstoffatomen bedeuten,
und ihren pharmazeutisch annehmbaren Säureadditionssalzen, dadurch gekennzeichnet, dass man

a) eine Verbindung der allgemeinen Formel

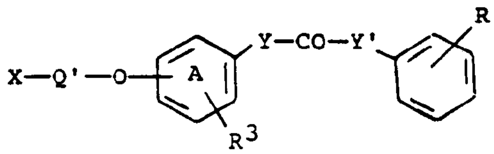

II

worin X eine Abgangsgruppe bedeutet, und A, Q', Y, Y', $R^3$ und R obige Bedeutung besitzen, mit einem Amin der allgemeinen Formel $HNR^1R^2$, worin $R^1$ und $R^2$ obige Bedeutung besitzen, umsetzt, oder

b) eine Verbindung der allgemeinen Formel

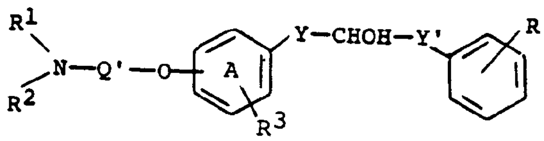

III.

worin A, $R^1$, $R^2$, $R^3$, Q', Y, Y' und R obige Bedeutung besitzen, oxidiert, oder

c) eine Verbindung der allgemeinen Formel

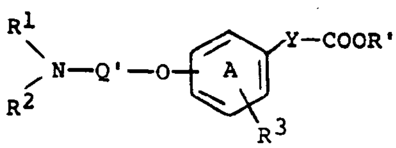

IV

worin R' niederes Alkyl bedeutet, und A, $R^1$, $R^2$, $R^3$, Q' und Y obige Bedeutung besitzen, mit einer Verbindung der allgemeinen Formel

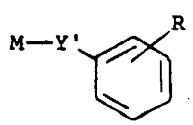

V

worin M -MgCl, -MgBr, -MgJ oder -Li bedeutet, und Y' und R obige Bedeutung besitzen, umsetzt, oder

d) eine Verbindung der allgemeinen Formel

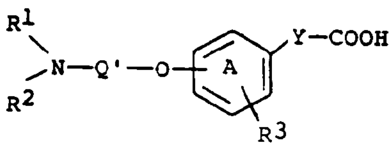

VI

worin A, $R^1$, $R^2$, $R^3$, Q' und Y obige Bedeutung besitzen, in Form eines reaktiven Derivates in Gegenwart einer Lewis-Säure mit einer Verbindung der allgemeinen Formel

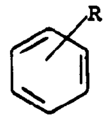

VII

worin R obige Bedeutung besitzt,
umsetzt, oder
e) eine Verbindung der allgemeinen Formel

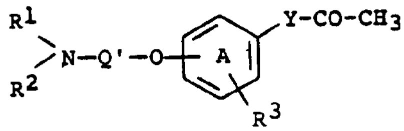

VIII

worin A, $R^1$, $R^2$, $R^3$, Q' und Y obige Bedeutung besitzen,
in Gegenwart einer Base mit einer Verbindung der allgemeinen Formel

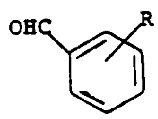

IX

worin R obige Bedeutung besitzt,
umsetzt, oder
f) eine Verbindung der allgemeinen Formel

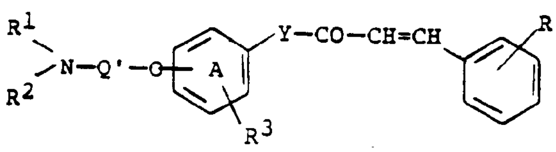

I'

worin A, $R^1$, $R^2$, $R^3$, Q', Y und R obige Bedeutung besitzen,
hydriert, oder
g) eine Verbindung der allgemeinen Formel

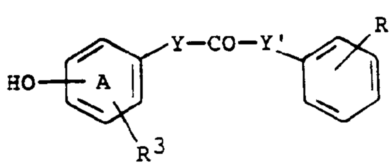

X

worin A, R, $R^3$, Y und Y' obige Bedeutung besitzen,
in Gegenwart von Triphenxylphosphin und einem Azodicarbonsäure-di(niederem Alkylester) mit einer Verbindung der allgemeinen Formel

$R^1R^2N$-Q'-OH XI

worin $R^1$, $R^2$ und Q' obige Bedeutung besitzen,
umsetzt, und
h) eine erhaltene Verbindung der Formel Ia erwünschtenfalls in ein pharmazeutisch annehmbares Säureadditionssalz überführt.

4. Verfahren nach Anspruch 3, worin $R^1$ und $R^2$ je Wasserstoff oder niederes Alkyl oder zusammen geradkettiges Alkylen mit 2 bis 4 Kohlenstoffatomen, $R^3$ Wasserstoff und Q' Alkylen mit 5 bis 11 Kohlenstoffatomen und mindestens 5 Kohlenstoffatomen zwischen den zwei freien Valenzen bedeuten, und das Symbol R bedeutet, dass der Ring unsubstituiert oder durch, Trifluormethyl, niederes Alkyl oder niederes Alkoxy substituiert ist.

5. Verfahren nach Anspruch 3 oder 4, worin Q' unverzweigtes Alkylen mit 5 bis 7 Kohlenstoffatomen bedeutet.

6. Verfahren nach Anspruch 3 oder 5, worin $R^1$ und $R^2$ je $C_{1-4}$-Alkyl oder $C_{3-4}$-Alkenyl oder zusammen $C_{3-4}$-Alkylen bedeuten.

7. Verfahren nach einem der Ansprüche 3 bis 6, worin die Gruppe $R^1R^2N$-Q'-O- an die 4-Stellung des mit A bezeichneten Ringes gebunden ist.

8. Verfahren nach einem der Ansprüche 3 bis 7, worin Y eine direkte Bindung oder die Gruppe $-CH_2-$ bedeutet.

9. Verfahren nach Anspruch 8, worin Y eine direkte Bindung bedeutet.

10. Verfahren nach einem der Ansprüche 3 bis 9, worin Y' eine direkte Bindung oder die Gruppe $-CH_2-$, $-CH_2CH_2-$ oder $-CH=CH-$ bedeutet.

11. Verfahren nach Anspruch 10, worin Y' eine direkte Bindung oder die Gruppe $-CH_2-$ bedeutet.

12. Verfahren nach einem der Ansprüche 3 bis 11, worin das Symbol R bedeutet, dass der Ring unsubstituiert oder durch niederes Alkyl mono- oder disubstituiert ist.

13. Verfahren nach Anspruch 3, dadurch gekennzeichnet, dass man
    4-[(6-(Dimethylamino)hexyl)oxy]-2-phenylacetophenon,
    4-[(6-(Dimethylamino)hexyl)oxy]benzophenon,
    4'-[(6-(Diäthylamino)hexyl)oxy]-3-phenylpropiophenon,
    4-[(6-(Dimethylamino)hexyl)oxy]-3-phenylpropiophenon,
    4'-[(6-(Dimethylamino)hexyl)oxy]-3-phenylacrylophenon,
    4-[(6-(Dimethylamino)hexyl)oxy]benzophenon,
    4-[(6-(1-Azetidinyl)hexyl)oxy]benzophenon,
    4-[(6-(1-Pyrrolidinyl)hexyl)oxy]benzophenon,
    4-[4-[(6-(Dimethylamino)hexyl)oxy]phenyl]acetophenon,
    4-[(7-(Dimethylamino)heptyl)oxy]benzophenon oder
    4-[(5-(Dimethylamino)pentyl)oxy]benzophenon
    herstellt.

14. Verfahren nach Anspruch 3, dadurch gekennzeichnet, dass man
    4-[[6-(Allylmethylamino)hexyl]oxy]-2-phenylacetophenon,
    trans-4-[[4-(Allylmethylamino)-2-butenyl]oxy]benzophenon,
    4-[[6-(Allylmethylamino)hexyl]oxy]-3-methylbenzophenon,
    oder
    4-[[6-(Allylmethylamino)hexyl]oxy]-3-chlorbenzophenon
    herstellt.

15. Verfahren zur Herstellung von Arzneimitteln, insbesondere von antifungal wirksamen Mitteln, dadurch gekennzeichnet, dass man eine Verbindung der in Anspruch 2 oder 3 definierten Formel Ia oder ein pharmazeutisch annehmbares Säureadditionssalz davon, und gegebenenfalls eine andere antifungal wirksame Substanz, welche die Sterol-Biosynthese hemmt, zusammen mit einem therapeutisch inerten Träger in eine galenische Darreichungsform bringt.

16. Verwendung von Verbindungen der in Anspruch 3 definierten Formel Ia und von pharmazeutisch annehmbaren Säureadditionssalzen davon gegebenenfalls in Kombination mit anderen antifungal wirksamen Substanzen, welche die Sterol-Biosynthese hemmen, für die Herstellung von antifungal wirksamen Mitteln.

## Claims

### Claims for the following Contracting States : AT, BE, CH, DE, DK, FR, GB, IT, LI, LU, NL, SE

1. The use of compounds of the general formula

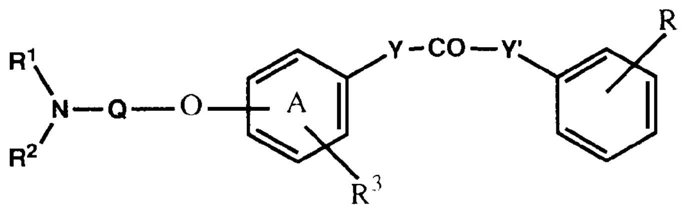

I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q-O- is attached to the 3- or 4-position of the ring denoted by A and the symbol R signifies that the ring is unsubstituted or is mono- or multiply-substituted by halogen, trifluoromethyl, cyano, nitro, lower alkyl and/or lower alkoxy,
and of their pharmaceutically acceptable acid addition salts, optionally in combination with antifungally-active substances which inhibit sterol biosynthesis, for the manufacture of antifungally-active medicaments.

2. Compounds of the general formula

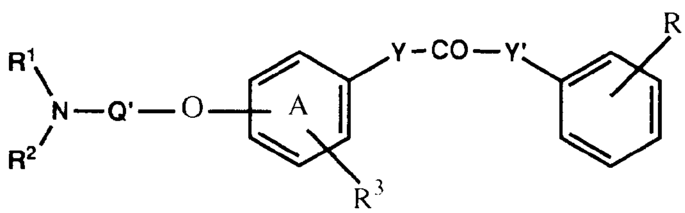

Ia

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q' signifies alkylene with 5 to 11 carbon atoms and at least 5 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q'-O- is attached to the 3- or 4-position of the ring denoted by A and the symbol R signifies that the ring is unsubstituted or is mono- or multiply-substituted by trifluoromethyl, cyano, lower alkyl and/or lower alkoxy,
and their pharmaceutically acceptable acid addition salts for use as therapeutically active substances, especially as antifungally-active substances.

3. Compounds of the general formula

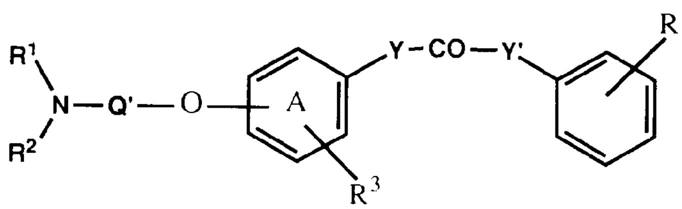

Ia

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q' signifies alkylene with 5 to 11 carbon atoms and at least 5 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q'-O- is attached to the 3- or 4-position of the ring denoted by A and the symbol R signifies that the ring is unsubstituted or is mono- or multiply-substituted by trifluoromethyl, cyano, lower alkyl and/or lower alkoxy, with the proviso that Y and Y' do not simultaneously signify a direct bond when Q' signifies alkylene with 5 carbon atoms and $R^1$ and $R^2$ simultaneously signify lower alkyl with more than 2 carbon atoms,
and their pharmaceutically acceptable acid addition salts.

**4.** Compounds according to claim 2 or 3, wherein $R^1$ and $R^2$ each signify hydrogen or lower alkyl or together signify straight-chain alkylene with 2 to 4 carbon atoms, $R^3$ signifies hydrogen and Q' signifies alkylene with 5 to 11 carbon atoms and at least 5 carbon atoms between the two free valencies and the symbol R signifies that the ring is unsubstituted or is substituted by trifluoromethyl, lower alkyl or lower alkoxy.

**5.** Compounds according to any one of claims 2 to 4, wherein Q' signifies unbranched alkylene with 5 to 7 carbon atoms.

**6.** Compounds according to any one of claims 2, 3 and 5, wherein $R^1$ and $R^2$ each signify $C_{1-4}$-alkyl or $C_{3-4}$-alkenyl or together signify $C_{3-4}$-alkylene.

**7.** Compounds according to any one of claims 2 to 6, wherein the group $R^1R^2N$-Q'-O- is attached to the 4-position of the ring denoted by A.

**8.** Compounds according to any one of claims 2 to 7, wherein Y signifies a direct bond or the group $-CH_2-$.

**9.** Compounds according to claim 8, wherein Y signifies a direct bond.

**10.** Compounds according to any one of claims 2 to 9, wherein Y' signifies a direct bond or the group $-CH_2-$, $-CH_2CH_2-$ or -CH=CH-.

**11.** Compounds according to claim 10, wherein Y' signifies a direct bond or the group $-CH_2-$.

**12.** Compounds according to any one of claims 2 to 11, wherein the symbol R signifies that the ring is unsubstituted or is mono- or disubstituted by lower alkyl.

**13.** 4-[(6-(Dimethylamino)hexyl)oxy]-2-phenylacetophenone,
4-[(6-(dimethylamino)hexyl)oxy]benzophenone,
4'-[(6(diethylamino)hexyl)oxy]-3-phenylpropiophenone,
4-[(6-(dimethylamino)hexyl)oxy]-3-phenylpropiophenone,
4'-[(6-(dimethylamino)hexyl)oxy]-3-phenylacrylophenone,
4-[(6-(dimethylamino)hexyl)oxy]benzophenone,
4-[(6-(1 -azetidinyl)hexyl)oxy]benzophenone,
4-[(6-(1-pyrrolidinyl)hexyl)oxy]benzophenone,
4-[4-[(6-(dimethylamino)hexyl)oxy]phenyl]acetophenone,
4-[(7-(dimethylamino)heptyl)oxy]benzophenone or
4-[(5-(dimethylamino)pentyl)oxy]benzophenone as the compound according to claim 2, 3 or 4.

**14.** 4-[[6-(Allylmethylamino)hexyl]oxy]-2-phenylacetophenone,
trans-4-[[4-(allylmethylamino)-2-butenyl]oxy]benzophenone,
4-[[6-(allylmethylamino)hexyl]oxy]-3-methylbenzophenone or
4-[[6-(allylmethylamino)hexyl]oxy]-3-chlorobenzophenone as the compound according to claim 2 or 3.

**15.** A process for the manufacture of compounds according to any one of claims 3 to 14 and of pharmaceutically acceptable acid addition salts thereof, characterized by
a) reacting a compound of the general formula

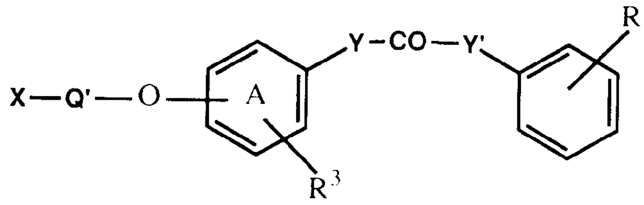

II

wherein X signifies a leaving group and A, Q', Y, Y', $R^3$ and R have the significance given in claim 3,

with an amine of the general formula $HNR^1R^2$, wherein $R^1$ and $R^2$ have the significance given in claim 3, or

b) oxidizing a compound of the general formula

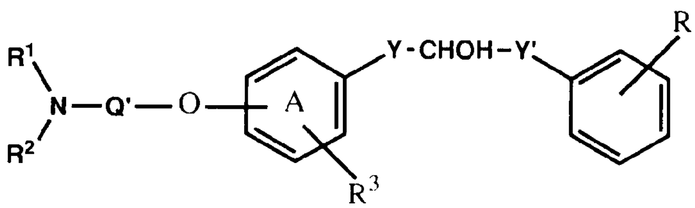

III

wherein A, $R^1$, $R^2$, $R^3$, Q', Y, Y' and R have the significance given in claim 3,

or

c) reacting a compound of the general formula

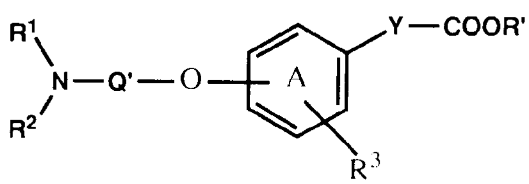

IV

wherein R' signifies lower alkyl and A, $R^1$, $R^2$, $R^3$, Q' and Y have the significance given in claim 3,

with a compound of the general formula

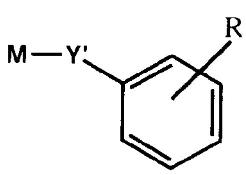

V

wherein M signifies -MgCl, -MgBr, -MgI or -Li and Y' and R have the significance given in claim 3,

or

d) reacting a compound of the general formula

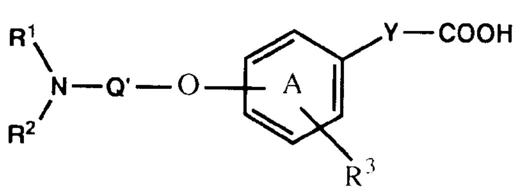

VI

wherein A, $R^1$, $R^2$, $R^3$, Q' and Y have the significance given in claim 3,

in the form of a reactive derivative in the presence of a Lewis acid with a compound of the general formula

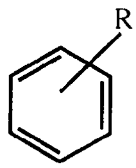

VII

wherein R has the significance given in claim 3,

or

e) reacting a compound of the general formula

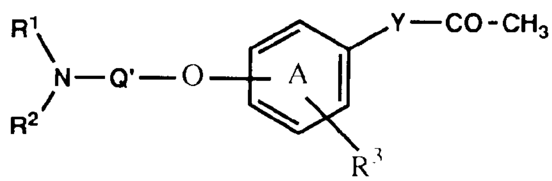

VIII

wherein A, $R^1$, $R^2$, $R^3$, Q' and Y have the significance given in claim 3,

in the presence of a base with a compound of the general formula

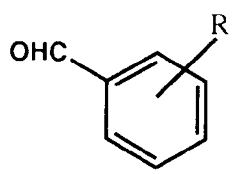

IX

wherein R has the significance given in claim 3,

or

f) hydrogenating a compound of the general formula

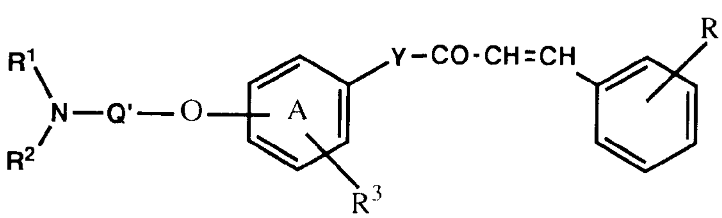

I'

wherein A, $R^1$, $R^2$, $R^3$, Q', Y and R have the significance given in claim 3,

or

g) reacting a compound of the general formula

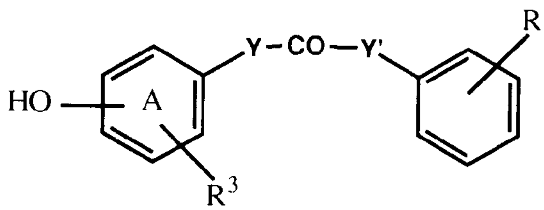

X

wherein A, R, $R^3$, Y and Y' have the significance given in claim 3, in the presence of triphenylphosphine and a di(lower alkyl) azodicarboxylate with a compound of the general formula

$R^1R^2N$-Q'-OH XI

wherein $R^1$, $R^2$ and Q' have the significance given in claim 3, and

h) if desired, converting a compound of formula Ia obtained into a pharmaceutically acceptable acid addition salt.

16. A medicament containing a compound in accordance with any one of claims 2 to 14 and a therapeutically inert carrier.

17. An antifungally-active medicament containing a compound in accordance with any one of claims 2 to 14 and a therapeutically inert carrier.

18. A medicament according to claim 16 or 17, which contains another antifungally-active substance which inhibits sterol biosynthesis as an additional active ingredient.

19. The use of compounds in accordance with any one of claims 2 to 14, optionally in combination with other antifungally-active substances which inhibit sterol biosynthesis, for the manufacture of antifungally-active medicaments.

**Claims for the following Contracting States : ES, GR**

1. The use of compounds of the general formula

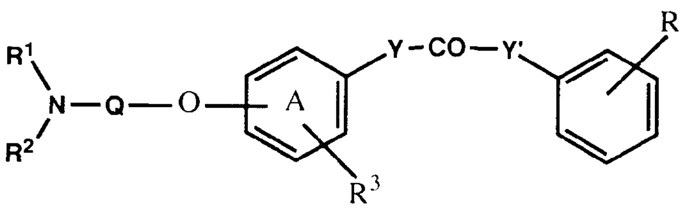

I

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q signifies alkylene or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q-O- is attached to the 3- or 4-position of the ring denoted by A and the symbol R signifies that the ring is unsubstituted or is mono- or multiply-substituted by halogen, trifluoromethyl, cyano, nitro, lower alkyl and/or lower alkoxy,
and of their pharmaceutically acceptable acid addition salts, optionally in combination with antifungally-active substances which inhibit sterol biosynthesis, for the manufacture of antifungally-active medicaments.

2. The use of compounds of the general formula

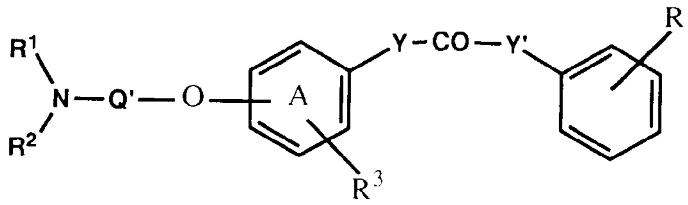

Ia

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q' signifies alkylene with 5 to 11 carbon atoms and at least 5 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q'-O- is attached to the 3- or 4-position of the ring denoted by A and the symbol R signifies that the ring is unsubstituted or is mono- or multiply-substituted by halogen, trifluoromethyl, cyano, nitro, lower alkyl and/or lower alkoxy, and of their pharmaceutically acceptable acid addition salts optionally in combination with antifungally-active substances which inhibit sterol biosynthesis, for the manufacture of antifungally-active medicaments.

3. A process for the manufacture of compounds of the general formula

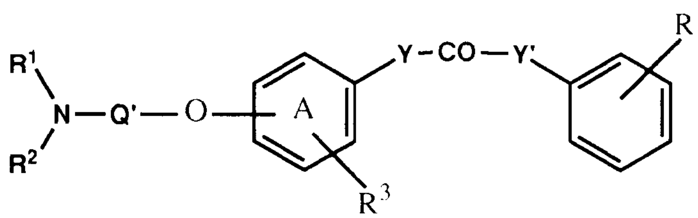

Ia

wherein $R^1$ and $R^2$ each signify hydrogen, lower alkyl or lower alkenyl or together signify straight-chain alkylene with 2 to 4 carbon atoms, $R^3$ signifies hydrogen, halogen or lower alkyl, Q' signifies alkylene with 5 to 11 carbon atoms and at least 5 carbon atoms between the two free valencies or alkenylene with 4 to 11 carbon atoms and at least 4 carbon atoms between the two free valencies and Y and Y' each signify a direct bond or the group $-CH_2-$, $-CH_2CH_2-$, -CH=CH- or -C≡C-, the group $R^1R^2N$-Q'-O- is attached to the 3- or 4-position of the ring denoted by A and the symbol R signifies that the ring is unsubstituted or is mono- or multiply-substituted by trifluoromethyl, cyano, lower alkyl and/or lower alkoxy, with the proviso that Y and Y' do not simultaneously signify a direct bond when Q' signifies alkylene with 5 carbon atoms and $R^1$ and $R^2$ simultaneously signify lower alkyl with more than 2 carbon atoms,
and of their pharmaceutically acceptable acid addition salts,
characterized by

a) reacting a compound of the general formula

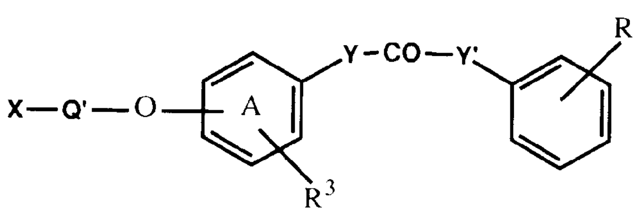

II

wherein X signifies a leaving group and A, Q', Y, Y', $R^3$ and R have the above significance, with an amine of the general formula $HNR^1R^2$, wherein $R^1$ and $R^2$ have the above significance, or

b) oxidizing a compound of the general formula

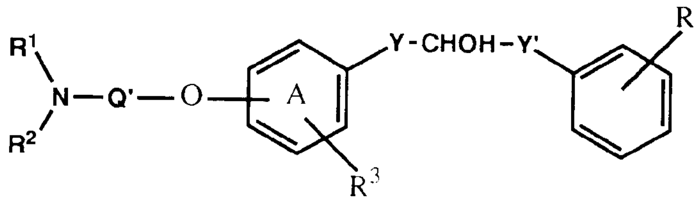

III

wherein A, $R^1$, $R^2$, $R^3$, Q', Y, Y' and R have the above significance,

or

c) reacting a compound of the general formula

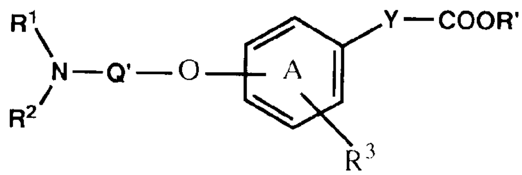

IV

wherein R' signifies lower alkyl and A, $R^1$, $R^2$, $R^3$, Q' and Y have the above significance, with a compound of the general formula

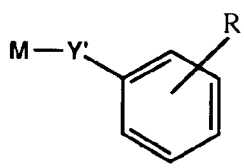

V

wherein M signifies -MgCl, -MgBr, -MgI or -Li and Y' and R have the above significance,

or

d) reacting a compound of the general formula

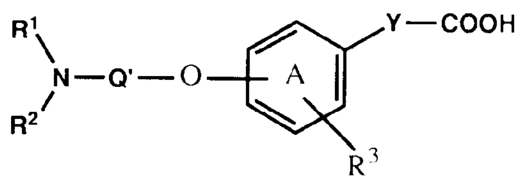

VI

wherein A, $R^1$, $R^2$, $R^3$, Q' and Y have the above significance, in the form of a reactive derivative in the presence of a Lewis acid with a compound of the general formula

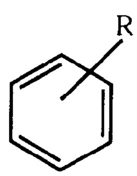

VII

wherein R has the above significance,

or

e) reacting a compound of the general formula

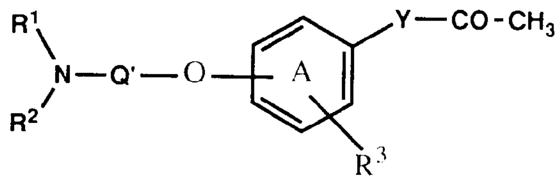

VIII

wherein A, $R^1$, $R^2$, $R^3$, Q' and Y have the above significance,
in the presence of a base with a compound of the general formula

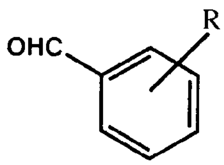

IX

wherein R has the above significance,
or
f) hydrogenating a compound of the general formula

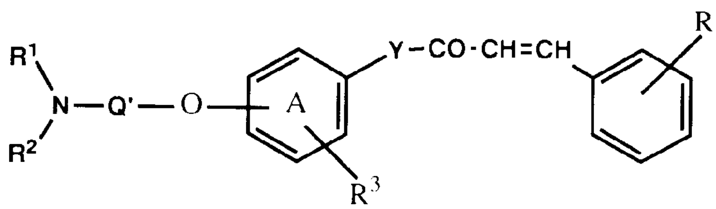

I'

wherein A, $R^1$, $R^2$, $R^3$, Q', Y and R have the above significance,
or
g) reacting a compound of the general formula

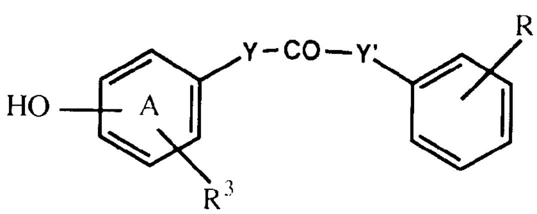

X

wherein A, R, $R^3$, Y and Y' have the above significance,
in the presence of triphenylphosphine and a di(lower alkyl) azodicarboxylate with a compound of the general formula

$$R^1R^2N\text{-}Q'\text{-}OH \qquad XI$$

wherein $R^1$, $R^2$ and Q' have the above significance,
and
h) if desired, converting a compound of formula Ia obtained into a pharmaceutically acceptable acid addition salt.

**4.** A process according to claim 3, wherein $R^1$ and $R^2$ each signify hydrogen or lower alkyl or together signify straight-chain alkylene with 2 to 4 carbon atoms, $R^3$ signifies hydrogen and Q' signifies alkylene with 5 to 11 carbon atoms and at least 5 carbon atoms between the two free valencies and the symbol R signifies that the ring is unsubstituted or is substituted by trifluoromethyl, lower alkyl or lower alkoxy.

5. A process according to claim 3 to 4, wherein Q' signifies unbranched alkylene with 5 to 7 carbon atoms.

6. A process according to claim 3 or 5, wherein $R^1$ and $R^2$ each signify $C_{1-4}$-alkyl or $C_{3-4}$-alkenyl or together signify $C_{3-4}$-alkylene.

7. A process according to any one of claims 3 to 6, wherein the group $R^1R^2N$-Q'-O- is attached to the 4-position of the ring denoted by A.

8. A process according to any one of claims 3 to 7, wherein Y signifies a direct bond or the group $-CH_2-$.

9. A process according to claim 8, wherein Y signifies a direct bond.

10. A process according to any one of claims 3 to 9, wherein Y' signifies a direct bond or the group $-CH_2-$, $-CH_2CH_2-$ or -CH=CH-.

11. A process according to claim 10, wherein Y' signifies a direct bond or the group $-CH_2-$.

12. A process according to any one of claims 3 to 11, wherein the symbol R signifies that the ring is unsubstituted or is mono- or disubstituted by lower alkyl.

13. A process according to claim 3, characterized in that
4-[(6-(dimethylamino)hexyl)oxy]-2-phenylacetophenone,
4-[(6-(dimethylamino)hexyl)oxy]benzophenone,
4'-[(6-(diethylamino)hexyl)oxy]-3-phenylpropiophenone,
4-[(6-(dimethylamino)hexyl)oxy]-3-phenylpropiophenone,
4'-[(6-(dimethylamino)hexyl)oxy]-3-phenylacrylophenone,
4-[(6-(dimethylamino)hexyl)oxy]benzophenone,
4-[(6-(1-azetidinyl)hexyl)oxy]benzophenone,
4-[(6-(1-pyrrolidinyl)hexyl)oxy]benzophenone,
4-[4-[(6-(dimethylamino)hexyl)oxy]phenyl]acetophenone,
4-[(7-(dimethylamino)heptyl)oxy]benzophenone or
4-[(5-(dimethylamino)pentyl)oxy]benzophenone
is manufactured.

14. A process according to claim 3, characterized in that
4-[[6-(allylmethylamino)hexyl]oxy]-2-phenylacetophenone,
trans-4-[[4-(allylmethylamino)-2-butenyl]oxy]benzophenone,
4-[[6-(allylmethylamino)hexyl]oxy]-3-methylbenzophenone
or
4-[[6-(allylmethylamino)hexyl]oxy]-3-chlorobenzophenone
is manufactured.

15. A process for the manufacture of medicaments, especially of antifungally-active medicaments, characterized by bringing a compound of formual Ia defined in claim 2 or 3 or a pharmaceutically acceptable acid addition salt thereof and, if desired, another antifungally-active substance which inhibits sterol biosynthesis into a galenical dosage form together with a therapeutically inert carrier.

16. The use of compounds of formula Ia defined in claim 3 and of pharmaceutically acceptable acid addition salts thereof, optionally in combination with other antifungally-active substances which inhibit sterol biosynthesis, for the manufacture of antifungally-active medicaments.

## Revendications

### Revendications pour les Etats contractants suivants : AT, BE, CH, DE, DK, FR, GB, IT, LI, LU, NL, SE

1. Utilisation des composés de formule générale

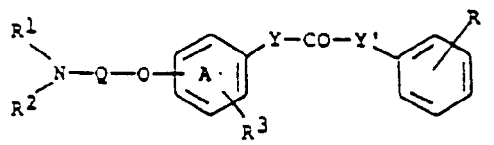

I

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q représente un groupe alkylène ou alcénylène contenant 4 à 11 atomes de carbone et au moins 4 atomes de carbone entre les deux valences libres, et Y et Y' représentent chacun une liaison directe ou un groupe $-CH_2-$, $-CH_2CH_2-$, -CH=CH- ou -C≡C-, le groupe $R^1R^2N$-Q-O- est relié en position 3 ou 4 du cycle A et le symbole R indique que le cycle est non substitué ou porte un ou plusieurs substituants halogéno, trifluorométhyle, cyano, nitro, alkyle inférieur et/ou alcoxy inférieur,
et de leurs sels formés par addition avec des acides acceptables pour l'usage pharmaceutique, éventuellement en combinaison avec des substances à activité antifongique qui inhibent la biosynthèse des stérols, pour la préparation de produits à activité antifongique.

2. Composés de formule générale

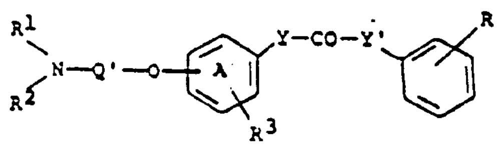

Ia

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q' représente un groupe alkylène contenant 5 à 11 atomes de carbone et au moins 5 atomes de carbone entre les deux valences libres, ou un groupe alcénylène contenant 4 à 11 atomes de carbone et au moins 4 atomes de carbone entre les deux valences libres, et Y et Y' représentent chacun une liaison directe ou un groupe $-CH_2-$, $-CH_2CH_2-$, -CH=CH- ou -C≡C-, le groupe $R^1R^2N$-Q'-O- est relié en position 3 ou 4 du cycle A et le symbole R indique que le cycle est non substitué ou porte un ou plusieurs substituants trifluorométhyle, cyano, alkyle inférieur et/ou alcoxy inférieur,
et leurs sels formés par addition avec des acides acceptables pour l'usage pharmaceutique, pour l'utilisation en tant que substances thérapeutiques actives, en particulier en tant que substances à activité antifongique.

3. Composés de formule générale

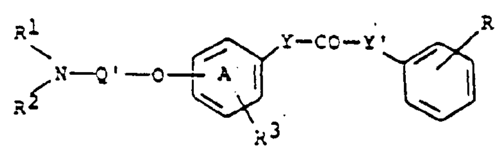

Ia

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q' représente un groupe alkylène contenant 5 à 11 atomes de carbone et au moins 5 atomes de carbone entre les deux valences libres, ou un groupe alcénylène contenant 4 à 11 atomes de carbone et au moins 4 atomes de carbone entre les deux valences libres, et Y et Y' représentent chacun une liaison directe ou un groupe $-CH_2-$, $-CH_2CH_2-$, -CH=CH- ou -C≡C-, le groupe $R^1R^2N$-Q'-O- est relié en position 3 ou 4 du cycle A et le symbole R indique que le cycle est non substitué ou porte un ou plusieurs substituants trifluorométhyle, cyano, alkyle inférieur et/ou alcoxy inférieur, sous réserve que Y et Y' ne peuvent représenter tous deux une liaison directe lorsque Q' représente un groupe alkylène à 5 atomes de carbone et que $R^1$ et $R^2$ représentent tous deux des groupes alkyle inférieurs à plus de deux atomes de carbone,
et leurs sels formés par addition avec des acides acceptables pour l'usage pharmaceutique.

4. Composés selon revendication 2 ou 3, pour lesquels $R^1$ et $R^2$ représentent chacun l'hydrogène ou un groupe alkyle inférieur ou forment ensemble un groupe alkylène à chaîne droite en C2-C4, $R^3$ représente l'hydrogène et Q' un groupe alkylène contenant 5 à 11 atomes de carbone et au moins 5 atomes de carbone entre les deux valences libres, et le symbole R indique que le cycle est non substitué ou substitué par des groupes trifluorométhyle, alkyle inférieur ou alcoxy inférieur.

5. Composés selon une des revendications 2 à 4, pour lesquels Q' représente un groupe alkylène à chaîne droite en C5-C7.

6. Composés selon une des revendications 2, 3 et 5, pour lesquels $R^1$ et $R^2$ représentent chacun un groupe alkyle en C1-C4 ou alcényle en C3-C4 ou forment ensemble un groupe alkylène en C3-C4.

7. Composés selon une des revendications 2 à 6, pour lesquels le groupe $R^1R^2N$-Q'-O- est relié en position 4 du cycle A.

8. Composés selon une des revendications 2 à 7, pour lesquels Y représente une liaison directe ou le groupe $-CH_2-$.

9. Composés selon revendication 8, pour lesquels Y représente une liaison directe.

10. Composés selon une des revendications 2 à 9, pour lesquels Y' représente une liaison directe ou un groupe $-CH_2-$, $-CH_2CH_2-$ ou -CH=CH-.

11. Composés selon revendication 10, pour lesquels Y' représente une liaison directe ou un groupe $-CH_2-$.

12. Composés selon une des revendications 2 à 11, pour lesquels le symbole R indique que le cycle est non substitué ou mono- ou di-substitué par des groupes alkyle inférieurs.

13. La 4-[(6-(diméthylamino)-hexyl)-oxy]-2-phénylacétophénone,
la 4-[(6-(diméthylamino)-hexyl)-oxy]-benzophénone,
la 4'-[(6-(diéthylamino)-hexyl)-oxy]-3-phénylpropiophénone,
la 4-[6-(diméthylamino)-hexyl)-oxy]-3-phénylpropiophénone,
la 4'-[(6-(diméthylamino)-hexyl)-oxy]-3-phénylacrylophénone,
la 4-[(6-(diméthylamino)-hexyl)-oxy]-benzophénone,
la 4-[(6-(1-azétidinyl)-hexyl)-oxy]-benzophénone,
la 4-[(6-(1-pyrrolidinyl)-hexyl)-oxy]-benzophénone,
la 4-[4-|(6-(diméthylamino)-hexyl)-oxy]-phényl|-acétophénone,
la 4-[(7-(diméthylamino)-heptyl)-oxy]-benzophénone, ou
la 4-[(5-(diméthylamino)-pentyl)-oxy]-benzophénone,
en tant que composé selon revendication 2, 3 ou 4.

14. La 4-[[6-(allylméthylamino)-hexyl]-oxy]-2-phénylacétophénone,
la trans-4-[[4-(allylméthylamino)-2-buténył]-oxy]-benzophénone,
la 4-[[6-(allylméthylamino)-hexyl]-oxy]-3-méthylbenzophénone,
ou bien
la 4-[[6-(allylméthylamino)-hexyl]-oxy]-3-chlorobenzophénone
en tant que composé selon revendication 2 ou 3.

15. Procédé de préparation des composés selon une des revendications 3 à 14 et de leurs sels formés par addition avec des acides acceptables pour l'usage pharmaceutique, caractérisé en ce que
a) on fait réagir un composé de formule générale

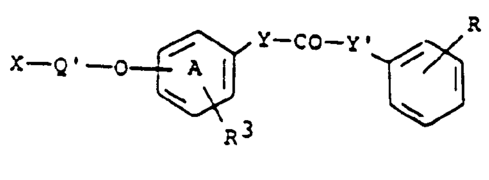

II

dans laquelle X représente un groupe éliminable et A, Q', Y, Y', $R^3$ et R ont les significations indiquées

dans la revendication 3,
avec une amine de formule générale $HNR^1R^2$ dans laquelle $R^1$ et $R^2$ ont les significations indiquées dans la revendication 3, ou bien
b) on oxyde un composé de formule générale

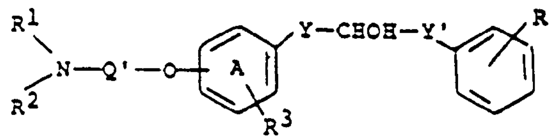

III

dans laquelle A, $R^1$, $R^2$, $R^3$, Q', Y, Y' et R ont les significations indiquées dans la revendication 3, ou bien
c) on fait réagir un composé de formule générale

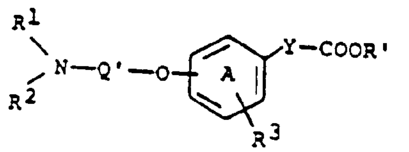

IV

dans laquelle R' représente un groupe alkyle inférieur et A, $R^1$, $R^2$, $R^3$, Q' et Y ont les significations indiquées dans la revendication 3,
avec un composé de formule générale

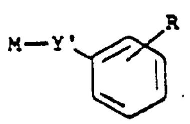

V

dans laquelle M représente -MgCl, -MgBr, -MgI ou -Li et Y' et R ont les significations indiquées dans la revendication 3, ou bien
d) on fait réagir un composé de formule générale

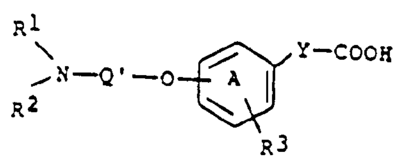

VI

dans laquelle A, $R^1$, $R^2$, $R^3$, Q' et Y ont les significations indiquées dans la revendication 3,
à l'état de dérivé réactif, en présence d'un acide de Lewis, avec un composé de formule générale

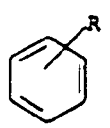

VII

dans laquelle R a les significations indiquées dans la revendication 3, ou bien
e) on fait réagir un composé de formule générale

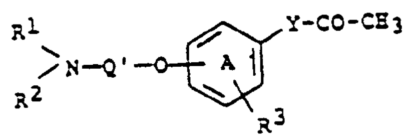

VIII

dans laquelle A, $R^1$, $R^2$, $R^3$, Q' et Y ont les significations indiquées dans la revendication 3, en présence d'une base, avec un composé de formule générale

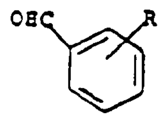

IX

dans laquelle R a les significations indiquées dans la revendication 3, ou bien
f) on hydrogène un composé de formule générale

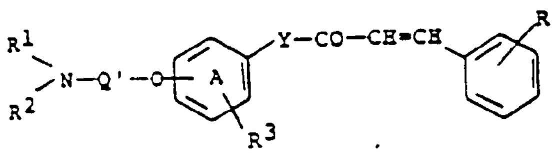

I'

dans laquelle A, $R^1$, $R^2$, $R^3$, Q', Y et R ont les significations indiquées dans la revendication 3, ou bien
g) on fait réagir un composé de formule générale

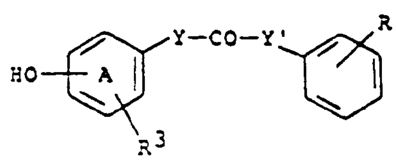

X

dans laquelle A, R, $R^3$, Y et Y' ont les significations indiquées dans la revendication 3,
en présence de la triphénylphosphine et d'un azodicarboxylate de dialkyle inférieur, avec un composé de formule générale

$R^1R^2N\text{-}Q'\text{-}OH$ XI

dans laquelle $R^1$, $R^2$ et Q' ont les significations indiquées dans la revendication 3, et
h) si on le désire, on convertit un composé ainsi obtenu, répondant à la formule Ia, en un sel par addition avec un acide acceptable pour l'usage pharmaceutique.

**16.** Médicament contenant un composé selon l'une des revendications 2 à 14 et un véhicule inerte du point de vue thérapeutique.

**17.** Agent antifongique actif contenant un composé selon une des revendications 2 à 14 et un véhicule inerte du point de vue thérapeutique.

**18.** Médicament selon revendication 16 ou 17, caractérisé en ce qu'il contient en tant que substance active additionnelle une autre substance à activité antifongique qui inhibe la biosynthèse des stérols.

**19.** Utilisation des composés selon l'une des revendications 2 à 14, éventuellement en combinaison avec d'autres substances à activité antifongique qui inhibent la biosynthèse des stérols, pour la préparation de produits à activité antifongique.

**Revendications pour les Etats contractants suivants : ES, GR**

**1.** Utilisation des composés de formule générale

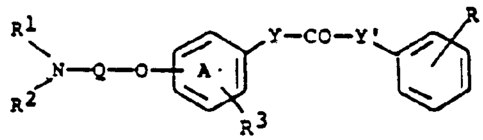

I

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q représente un groupe alkylène ou alcénylène contenant 4 à 11 atomes de carbone et au moins 4 atomes de carbone entre les deux valences libres,et Y et Y' représentent chacun une liaison directe ou un groupe $-CH_2-$, $-CH_2CH_2-$, -CH=CH- ou -C≡C-, le groupe $R^1R^2N$-Q-O- est relié en position 3 ou 4 du cycle A et le symbole R indique que le cycle est non substitué ou porte un ou plusieurs substituants halogéno, trifluorométhyle, cyano, nitro, alkyle inférieur et/ou alcoxy inférieur,
et de leurs sels formés par addition avec des acides acceptables pour l'usage pharmaceutique, éventuellement en combinaison avec des substances à activité antifongique qui inhibent la biosynthèse des stérols, pour la préparation de produits à activité antifongique.

**2.** utilisation des composés de formule générale

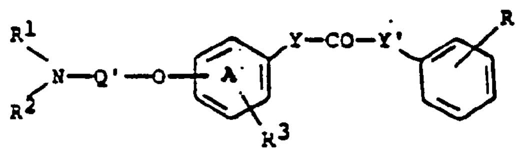

Ia

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q' représente un groupe alkylène contenant 5 à 11 atomes de carbone et au moins 5 atomes de carbone entre les deux valences libres, ou un groupe alcénylène contenant 4 à 11 atomes de carbone et au moins 4 atomes de carbone entre les deux valences libres, et Y et Y' représentent chacun une liaison directe ou un groupe $-CH_2-$, $CH_2CH_2-$, -CH=CH- ou -C≡C-, le groupe $R^1R^2N$-Q'-O- est relié en position 3 ou 4 du cycle A et le symbole R indique que le cycle est non substitué ou porte un plusieurs substituants trifluorométhyle, cyano, alkyle inférieur et/ou alcoxy inférieur,
et de leurs sels formés par addition avec des acides acceptables pour l'usage pharmaceutique, éventuellement en combinaison avec des substances à activité antifongique qui inhibent la biosynthèse des stérols, pour la préparation de produits à activité antifongique.

**3.** Procédé de préparation des composés de formule générale

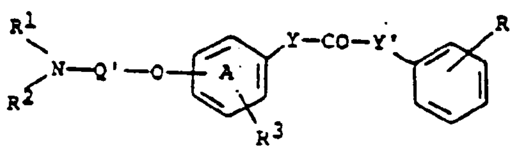

Ia

dans laquelle $R^1$ et $R^2$ représentent chacun l'hydrogène, un groupe alkyle inférieur ou alcényle inférieur ou forment ensemble un groupe alkylène à chaîne droite en C2-C4, $R^3$ représente l'hydrogène, un halogène ou un groupe alkyle inférieur, Q' représente un groupe alkylène contenant 5 à 11 atomes de carbone et au moins 5 atomes de carbone entre les deux valences libres, ou un groupe alcénylène contenant 4 à 11 atomes de carbone et au moins 4 atomes de carbone entre les deux valences libres, et Y et Y' représentent chacun une liaison directe ou un groupe $-CH_2-$, $-CH_2CH_2-$, -CH=CH- ou -C≡C-, le groupe $R^1R^2N$-Q'-O- est relié en position 3 ou 4 du cycle A et le symbole R indique que le cycle est non substitué ou porte un ou plusieurs substituants trifluorométhyle, cyano, alkyle inférieur et/ou alcoxy inférieur, sous réserve que Y et Y' ne peuvent représenter tous deux une liaison directe lorsque Q' représente un groupe alkylène à 5 atomes de carbone et que $R^1$ et $R^2$ représentent tous deux des groupes alkyle inférieurs à plus de deux atomes de carbone,
et leurs sels formés par addition avec des acides acceptables pour l'usage pharmaceutique,
caractérisé en ce que

a) on fait réagir un composé de formule générale

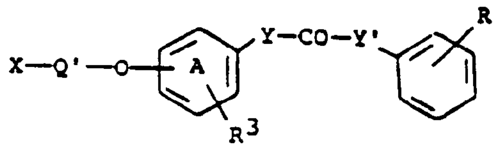

II

dans laquelle X représente un groupe éliminable et A, Q', Y, Y', $R^3$ et R ont les significations indiquées ci-dessus,
avec une amine de formule générale $HNR^1R^2$ dans laquelle $R^1$ et $R^2$ ont les significations indiquées ci-dessus, ou bien
b) on oxyde un composé de formule générale

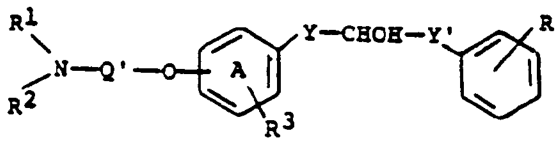

III

dans laquelle A, $R^1$, $R^2$, $R^3$, Q', Y, Y' et R ont les significations indiquées ci-dessus, ou bien
c) on fait réagir un composé de formule générale

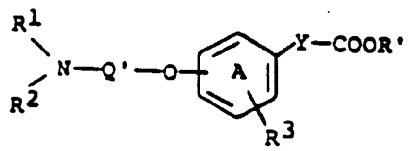

IV

dans laquelle R' représente un groupe alkyle inférieur et A, $R^1$, $R^2$, $R^3$, Q' et Y ont les significations indiquées ci-dessus,
avec un composé de formule générale

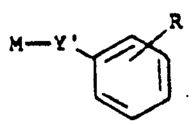

V

dans laquelle M représente -MgCl, -MgBr, -MgI ou -Li et Y' et R ont les significations indiquées ci-dessus, ou bien
d) on fait réagir un composé de formule générale

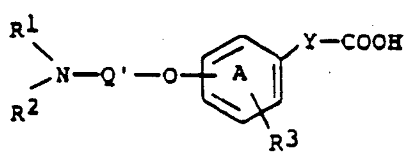

VI

dans laquelle A, $R^1$, $R^2$, $R^3$, Q' et Y ont les significations indiquées ci-dessus,
à l'état de dérivé réactif, en présence d'un acide de Lewis, avec un composé de formule générale

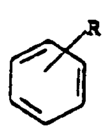

VII

dans laquelle R a les significations indiquées ci-dessus, ou bien
e) on fait réagir un composé de formule générale

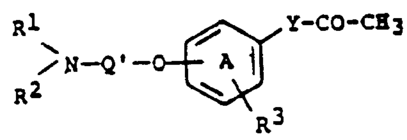

VIII

dans laquelle A, $R^1$, $R^2$, $R^3$, Q' et Y ont les significations indiquées ci-dessus,
en présence d'une base, avec un composé de formule générale

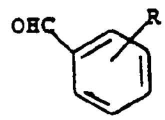

IX

dans laquelle R a les significations indiquées ci-dessus, ou bien
f) on hydrogène un composé de formule générale

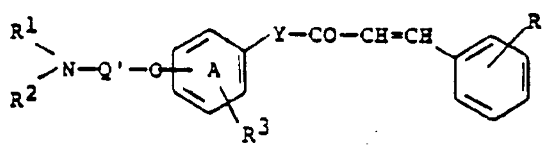

I'

dans laquelle A, $R^1$, $R^2$, $R^3$, Q', Y et R ont les significations indiquées ci-dessus, ou bien
g) on fait réagir un composé de formule générale

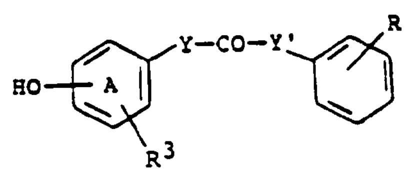

X

dans laquelle A, R, $R^3$, Y et Y' ont les significations indiquées ci-dessus,
en présence de la triphénylphosphine et d'un azodicarboxylate de dialkyle inférieur,
avec un composé de formule générale

$R^1R^2N$-Q'-OH XI

dans laquelle $R^1$, $R^2$ et Q' ont les significations indiquées ci-dessus, et
h) si on le désire, on convertit un composé ainsi obtenu, répondant à la formule Ia, en un sel par addition avec un acide acceptable pour l'usage pharmaceutique.

**4.** Procédé selon revendication 3, dans lequel $R^1$ et $R^2$ représentent chacun l'hydrogène ou un groupe alkyle inférieur ou forment ensemble un groupe alkylène à chaîne droite en C2-C4, $R^3$ représente l'hydrogène et Q' un groupe alkylène contenant 5 à 11 atomes de carbone et au moins 5 atomes de carbone entre les deux valences libres, et le symbole R indique que le cycle est non substitué ou substitué par des groupes trifluorométhyle, alkyle inférieur ou alcoxy inférieur.

**5.** Procédé selon revendication 3 ou 4, dans lequel Q' représente un groupe alkylène à chaîne droite en C5-C7.

**6.** Procédé selon revendication 3 ou 5, dans lequel $R^1$ et $R^2$ représentent chacun un groupe alkyle en C1-C4 ou alcényle en C3-C4 ou forment ensemble un groupe alkylène en C3-C4.

**7.** Procédé selon une des revendications 3 à 6, dans lequel le groupe $R^1R^2N$-Q'-O- est relié en position 4

du cycle A.

8. Procédé selon une des revendications 3 à 7, dans lequel Y représente une liaison directe ou le groupe $-CH_2-$.

9. Procédé selon revendication 8, dans lequel Y représente une liaison directe.

10. Procédé selon une des revendications 3 à 9, dans lequel Y' représente une liaison directe ou un groupe $-CH_2-$, $-CH_2CH_2-$ ou -CH=CH-.

11. Procédé selon revendication 10, dans lequel Y' représente une liaison directe ou le groupe $-CH_2-$.

12. Procédé selon une des revendications 3 à 11, dans lequel le symbole R indique que le cycle est non substitué ou mono- ou di-substitué par des groupes alkyle inférieurs.

13. Procédé selon revendication 3, caractérisé en ce que l'on prépare
la 4-[(6-(diméthylamino)-hexyl)-oxy]-2-phénylacétophénone,
la 4-[(6-(diméthylamino)-hexyl)-oxy]-benzophénone,
la 4'-[(6-(diéthylamino)-hexyl)-oxy]-3-phénylpropiophénone,
la 4-[(6-(diméthylamino)-hexyl)-oxy]-3-phénylpropiophénone,
la 4'-[(6-(diméthylamino)-hexyl)-oxy]-3-phénylacrylophénone,
la 4-[(6-(diméthylamino)-hexyl)-oxy]-benzophénone,
la 4-[6-(1-azétidinyl)-hexyl)-oxy]-benzophénone,
la 4-[(6-(1-pyrrolidinyl)-hexyl)-oxy]-benzophénone,
la 4-[4-[(6-(diméthylamino)-hexyl)-oxy]-phényl]-acétophénone,
la 4-[(7-(diméthylamino)-heptyl)-oxy]-benzophénone, ou
la 4-[(5-(diméthylamino)-pentyl)-oxy]-benzophénone.

14. Procédé selon revendication 3, caractérisé en ce que l'on prépare
la 4-[[6-(allylméthylamino)-hexyl]-oxy]-2-phénylacétophénone,
la trans-4-[[4-(allylméthylamino)-2-butényl]oxy]-benzophénone,
la 4-[[6-(allylméthylamino)-hexyl]-oxy]-3-méthylbenzophénone,
ou bien
la 4-[[6-(allylméthylamino)-hexyll-oxy]-3-chlorobenzophénone.

15. Procédé de préparation de médicaments, en particulier de médicaments à activité antifongique, caractérisé en ce que l'on met sous une forme d'administration galénique un composé de formule Ia définie dans la revendication 2 ou 3 ou un sel d'un tel composé formé par addition avec un acide acceptable pour l'usage pharmaceutique, et le cas échéant une autre substance à activité antifongique qui inhibe la biosynthèse des stérols, avec un véhicule inerte du point de vue thérapeutique.

16. utilisation des composés de formule Ia définie dans la revendication 3 et de leurs sels formés par addition avec des acides acceptables pour l'usage pharmaceutique, éventuellement en combinaison avec d'autres substances à activité antifongique inhibant la biosynthèse des stérols, pour la préparation de produits à activité antifongique.